# EUROPÄISCHE PATENTANMELDUNG

(11) **EP 2 228 375 A1**
(43) Veröffentlichungstag der Anmeldung: **15.09.2010**
(21) Anmeldenummer: 10158963.8
(22) Anmeldetag: 03.11.2003
(51) Int. Cl.: C07D 473/04, A61P 5/50, A61K 31/522

(54) **Neue Xanthinderivate, deren Herstellung und deren Verwendung als Arzneimittel**

(30) Priorität: 08.11.2002 DE 10251927
(62) Teilanmeldung aus: 03788995.3
(71) Anmelder: Boehringer Ingelheim Pharma GmbH & Co. KG, 55216 Ingelheim am Rhein (DE)
(72) Erfinder: Himmelsbach, Frank, 88441 MITTELBIBERACH (DE); Langkopf, Elke, 88447 WARTHAUSEN (DE); Eckhardt, Matthias, 88400 BIBERACH (DE); Maier, Roland, 88400 BIBERACH (DE); Mark, Michael, 88400 BIBERACH (DE); Tadayyon, Mohammad, WATFORD, WD 17 4HU (GB); Lotz, Ralf, 88433 SCHEMMERHOFEN (DE)
(74) Vertreter: Hammann, Heinz

(57) **Zusammenfassung**

Die vorliegende Erfindung betrifft substituierte Xanthine der allgemeinen Formel 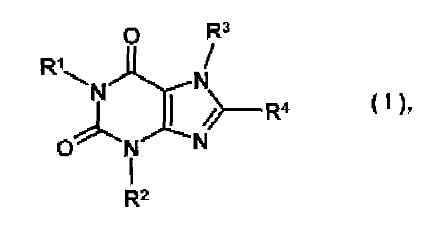 in der R¹ bis R⁴ wie in Anspruch 1 definiert sind, deren Tautomere, deren Stereoisomere, deren Gemische, deren Prodrugs und deren Salze, welche wertvolle pharmakologische Eigenschaften aufweisen, insbesondere eine Hemmwirkung auf die Aktivität des Enzyms Dipeptidylpeptidase-IV (DPP-IV).

## Beschreibung

Gegenstand der vorliegenden Erfindung sind neue substituierte Xanthine der allgemeinen Formel 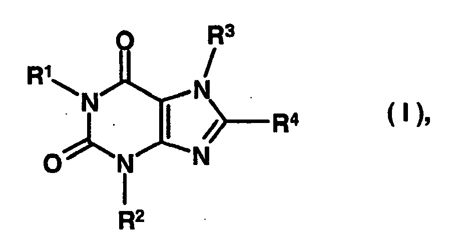 deren Tautomere, Enantiomere, Diastereomere, deren Gemische, deren Prodrugs und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Gegenstand der vorliegenden Erfindung sind somit die obigen Verbindungen der allgemeinen Formel I, welche wertvolle pharmakologische Eigenschaften aufweisen, die die pharmakologisch wirksamen Verbindungen enthaltenden Arzneimittel, deren Verwendung und Verfahren zu ihrer Herstellung.

In der obigen allgemeinen Formel I bedeuten

R¹ eine durch eine Gruppe Rₐ substituierte C₁₋₃-Alkylgruppe, wobei Rₐ eine 1,4-Dihydro-chinazolinyl- oder 3,4-Dihydro-chinazolinylgruppe, in der jeweils im Benzoteil
eine bis drei Methingruppen durch Stickstoffatome ersetzt sein können,
eine 3,4-Dihydro-isochinolinyl-, 1*H*-Benzo[*d*][1,2]oxazinyl-, 4*H*-Benzo[*e*][1,3]oxazinyl-, 4*H*-Benzo[*d*][1,3]oxazinyl- oder 2*H*-Benzo[1,4]oxazinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 4*H*-Benzo[*e*][1,3]thiazinyl-, 4*H*-Benzo[*d*][1,3]thiazinyl- oder 2*H-*Benzo[1,4]thiazinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann sowie ein Schwefelatom durch eine Sulfinyl- oder Sulfonylgruppe ersetzt sein kann,
eine 2-Oxo-2*H*-benzo[*e*][1,3]oxazinyl- oder 2,2-Dioxo-1*H-*benzo[*c*][1,2]thiazinylgruppe, in der jeweils im Benzoteil
eine bis drei Methingruppen durch Stickstoffatome ersetzt sein können,
eine 2,3-Dihydro-1*H*-benzo[*e*][1,4]diazepinyl-, 4,5-Dihydro-3*H*-benzo[*b*]-[1,4]diazepinyl- oder 5-Oxo-4,5-dihydro-3*H*-benzo[*e*][1,4]diazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 2,3-Dihydro-benzo[*f*][1,4]oxazepinyl- oder 2,3-Dihydro-benzo[*b*][1,4]oxazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 2,3-Dihydro-benzo[*f*][1,4]thiazepinyl- oder 2,3-Dihydro-benzo[*b*][1,4]thiazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann und ein Schwefelatom durch eine Sulfinyl- oder Sulfonylgruppe ersetzt sein kann,
eine 5-Oxo-4,5-dihydro-benzo[*f*][1,3,4]oxadiazepinylgruppe, in der im Benzoteil
eine bis drei Methingruppen durch Stickstoffatome ersetzt sein können,
eine 11*H*-Dibenzo[*b*,*e*]azepinyl- oder 5H-Dibenzo[*a*,*d*]cycloheptenylgruppe, in der jeweils
im Benzoteil eine bis drei Methingruppen durch Stickstoffatome ersetzt sein können und die Methylengruppe im Heterocyclylteil durch ein Sauerstoff- oder Schwefelatom, eine Carbonyl-, Sulfinyl-, Sulfonyl- oder eine durch Rₓ substituierte Iminogruppe ersetzt sein kann, wobei
Rₓ ein Wasserstoffatom oder eine C₁₋₄-Alkyl-, C₂₋₄-Alkenyl-, C₂₋₄-Alkinyl-, C₃₋₆-Cycloalkyl-, C₃₋₆-Cycloalkyl-C₁₋₃-alkyl-, Aryl-, Aryl-C₁₋₃-alkyl-, Hydroxy-C₂₋₄-clkyl-, C₁₋₃-Alkyloxy-C₂₋₄-alkyl-, C₃₋₆-Cyclo-alkyloxy-C₂₋₄-alkyl-, Amino-C₂₋₄-alkyl-, C₁₋₃-Alkylamino-C₂₋₄-alkyl, Di-(C₁₋₃-alkyl)-amino-C₂₋₄-alkyl-, C₁₋₃-Alkyl-carbonyl-, C₁₋₃-Alkyl-oxy-carbonyl-, C₁₋₃-Alkyloxy-carbonyl-C₁₋₃-alkyl-, Aryl-carbonyl-, C₁₋₃-Alkyl-sulfonyl- oder Aryl-sulfonylgruppe darstellt,
eine Phenanthridinyl-, 1,2,3,4-Tetrahydro-phenanthridinyl-, Benzo[*f*]chinoxalinyl-, 5*H*-Dibenzo[d,*f*][1,3]diazepinyl-, 5H-Benzo[*e*]pyrrolo[1,2-a][1,4]diazepinyl-, Thieno[3,2-b][1,4]benzoxazepinyl- oder eine 3-Oxo-2,3-dihydro-isoindol-1-ylidengruppe, in der jeweils
im Benzoteil eine bis drei Methingruppen durch Stickstoffatome ersetzt sein können,
eine Benzo[1,2,5]oxadiazolyl-, Dibenzofuranyl-, Indolizinyl-, 1*H*-Perimidinyl-, gruppe,
eine Pyrazolo[1,5-c]chinzolinylgruppe odereine Imidazo[2,1-a]isochinolinyl-Imidazo[1,2-a]isochinolinylgruppe
bedeutet, wobei die vorstehend erwähnten Reste Rₐ durch die Gruppen R¹⁰ bis R¹³ substituiert sein können und zusätzlich durch eine C₁₋₃-Alkylgruppe substituiert sein können und
R¹⁰ ein Wasserstoffatom,
ein Fluor-, Chlor-, Brom- oder Iodatom,
eine C₁₋₄-Alkyl-, Hydroxy-, oder C₁₋₄-Alkyloxygruppe,
eine Nitro-, Amino-, C₁₋₃-Alkylamino-, Di-(C₁₋₃-alkyl)amino-, Cyan-C₁₋₃-alkylamino-, [N-(Cyan-C₁₋₃-alkyl)-N-C₁₋₃-alkyl-amino]-, C₁₋₃-Alkyloxy-carbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
eine C₁₋₃-Alkyl-carbonylamino-, Arylcarbonylamino-, Aryl-C₁₋₃-alkyl-carbonylamino-, C₁₋₃-Alkyloxy-carbonylamino-, Aminocarbonylamino-,
C₁₋₃-Alkyl-aminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkylamino-sulfonylamino-, Di-(C₁₋₃-alkyl)amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonylamino-, Arylsulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-arylsulfonylamino-, oder N-(C₁₋₃-Alkyl)-aryl-C₁₋₃-alkyl-sulfonylaminogruppe,
eine 2-Oxo-imidazolidin-1-yl-, 2,4-Dioxo-imidazolidin-1-yl-, 2,5-Dioxo-imidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1-yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-,
Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, C₁₋₃-Alkyl-sulfanyl-C₁₋₃-alkyloxy-, C₁₋₃-Alkylsulfinyl-C₁₋₃-alkyloxy-, C₁₋₃-Alkyl-sultonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl- oder Trifluormethylsulfonylgruppe,
eine Sulfo-, Aminosulfonyl-, C₁₋₃-Alkyl-aminosulfonyl-, Di-(C₁₋₃-alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonylgruppe,
eine durch 1 bis 3 Fluoratome substituierte Methyl- oder Methoxygruppe, eine durch 1 bis 5 Fluoratome substituierte Ethyl- oder Ethoxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder Iodatom, eine C₁₋₃-Alkyl-, Trifluormethyl-, Hydroxy-, oder C₁₋₃-Alkyloxygruppe oder eine Cyangruppe, oder
R¹¹ zusammen mit R¹², sofern diese an benachbarte Kohlenstoffatome gebunden sind, auch eine Methylendioxy-, Difluormethylendioxy-, Ethylendioxy- oder eine geradkettige C₃₋₅-Alkylengruppe und
R¹³ ein Wasserstoffatom, ein Fluor-, Chlor- oder Bromatom, eine Trifluormethyl-, C₁₋₃-Alkyl- oder C₁₋₃-Alkyloxygruppe bedeuten,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃-₆-Cycloalkyl-C₁-₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonyl-C₁₋₂-alkylgruppe,
eine Heteroaryl-C₁₋₃-alkylgruppe,
eine Furanylcarbonylmethyl-, Thienylcarbonylmethyl-, Thiazolylcarbonylmethyl- oder Pyridylcarbonylmethylgruppe,
eine C₁-₄-Alkyl-carbonyl-C₁₋₂-alkyl-Gruppe,
eine C₃₋₆-Cycloalkyl-carbonyl-C₁₋₂-alkyl-Gruppe,
eine Aryl-A-C₁-₃-alkylgruppe, wobei A eine Sauerstoff- oder Schwefelatom, eine Imino-, C₁₋₃-Alkylimino-, Sulfinyl- oder Sulfonylgruppe bedeutet, eine durch eine Gruppe R_{b} substituierte C₁₋₄-Alkylgruppe, wobei
R_{b} eine Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet, oder eine durch eine Gruppe R_{c} substituierte C₂₋₄-Alkylgruppe, wobei
R_{c} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 3-Stellung des Xanthingerüstes isoliert ist, R³ eine C₃₋₈-Alkylgruppe,
eine durch eine Gruppe R_{d} substituierte C₁₋₃-Alkylgruppe, wobei
R_{d} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe, eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cydoalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cucloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piporidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder Iodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei, der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

Verbindungen der obigen allgemeinen Formel I, die einen oder mehrere in-vivo abspaltbare Reste enthalten, stellen sogenannte Prodrugs dar.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,
desweiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Journal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
Rq ein Wasserstoffatom, eine C₁₋₃-Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylamino-carbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxy-carbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkyloxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxycarbonyl-, C₁₋₃-Alkyloxy-CC₂₋₄-alkyloxy-C₂₋₄-alkyloxycarbonyl-, Rₚ-CO-O-(R_{q}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
Rₛ und Rₜ, die gleich oder verschieden sein können, Wasserstoffatome oder C₁₋₃-Alkylgruppen darstellen,
zu verstehen.

Desweiteren schließen die in den vor- und nachstehenden Definitionen erwähnten gesättigten Alkyl- und Alkyloxyteile, die mehr als 2 Kohlenstoffatome enthalten, soweit nichts Anderes erwähnt wurde, auch deren verzweigte Isomere wie beispielsweise die Isopropyl-, tert.Butyl-, Isobutylgruppe etc. ein.

Für R² kommt beispielsweise jeweils die Bedeutung eines Wasserstoffatoms, einer Methyl-, Ethyl-, Propyl-, 2-Propyl-, Butyl-, 2-Butyl-, 2-Methylpropyl-, 2-Propen-1-yl-, 2-Propin-1-yl-, Cyclopropylmethyl-, Benzyl-, 2-Phenylethyl-, Phenylcarbonylmethyl-, 3-Phenylpropyl-, 2-Hydroxyethyl-, 2-Methoxyethyl-, 2-Ethoxyethyl-, 2-(Dimethylamino)-ethyl-, 2-(Diethylamino)ethyl-, 2-(Pyrrolidino)ethyl-, 2-(Piperidino)ethyl-, 2-(Morpholino)ethyl-, 2-(Piperazino)ethyl-, 2-(4-Methylpiperazino)ethyl-, 3-Hydroxypropyl-, 3-Methoxypropyl-, 3-Ethoxypropyl-, 3-(Dimethylamino)propyl-, 3-(Diethylamino)propyl-, 3-(Pyrrolidino)propyl-, 3-(Piperidino)propyl-, 3-(Morpholino)propyl-,3-(Piperazino)-propyl-, 3-(4-Methylpiperazino)propyl-, Carboxymethyl-, (Methoxycarbonyl)methyl-, (Ethoxycarbonyl)methyl-, 2-Carboxyethyl-, 2-(Methoxycarbonyl)ethyl-, 2-(Ethoxycarbonyl)ethyl-, 3-Carboxypropyl-, 3-(Methoxycarbonyl)propyl-, 3-(Ethoxycarbonyl)-propyl-, (Aminocarbonyl)methyl-, (Methylaminocarbonyl)methyl-, (Dimethylaminocarbonyl)methyl-, (Pyrrolidinocarbonyl)methyl-, (Piperidinocarbonyl)methyl-, (Morpholinocarbonyl)methyl-, 2-(Aminocarbonyl)ethyl-, 2-(Methylaminocarbonyl)ethyl-, 2-(Dimethylaminocarbonyl)ethyl-, 2-(Pyrrolidinocarbonyl)ethyl-, 2-(Piperidinocarbonyl)-ethyl-, 2-(Morpholinocarbonyl)ethyl-, Cyanmethyl- oder 2-Cyanethylgruppe in Betracht.

Für R³ kommt beispielsweise die Bedeutung einer Methyl-, Ethyl-, Propyl-, 2-Propyl-, Butyl-, 2-Butyl-, 2-Methylpropyl-, Pentyl-, 2-Methylbutyl-, 3-Methylbutyl-, 2,2-Dimethylpropyl-, Cyclopropylmethyl-, (1-Methylcyclopropyl)methyl-, (2-Methylcyclopropyl)methyl-, Cyclobutylmethyl-, Cyclopentylmethyl-, Cyclohexylmethyl-, 2-(Cyclopropyl)ethyl-,
2-Propen-1-yl-, 2-Methyl-2-propen-1-yl-, 3-Phenyl-2-propen-1-yl-, 2-Buten-1-yl-, 4,4,4-Trifluor-2-buten-1-yl-, 3-Buten-1-yl-, 2-Chlor-2-buten-1-yl-, 2-Brom-2-buten-1-yl-, 3-Chlor-2-buten-1-yl-, 3-Brom-2-buten-1-yl-, 2-Methyl-2-buten-1-yl-, 3-Methyl-2-buten-1-yl-, 2,3-Dimethyl-2-buten-1-yl-, 3-Trifluormethyl-2-buten-1-yl-, 3-Methyl-3-buten-1-yl-,
1-Cyclopenten-1-ylmethyl-, (2-Methyl-1-cyclopenten-1-yl)methyl-, 1-Cyclohexen-1-ylmethyl-, 2-(1-Cyclopenten-1-yl)ethyl-, 2-Propin-1-yl-, 2-Butin-1-yl, 3-Butin-1-yl, Phenyl-, Methylphenyl-, Benzyl-, eine Fluorbenzyl-, Chlorbenzyl-, Brombenzyl-, Methylbenzyl-, Methoxybenzyl-, 1-Phenylethyl-, 2-Phenylethyl-, 3-Phenylpropyl-, 2-Furanylmethyl, 3-Furanylmethyl-, 2-Thienylmethyl- oder 3-Thienylmethylgruppe in Betracht.

Für R⁴ kommt beispielsweise die Bedeutung einer 3-Aminopyrrolidin-1-yl-, 3-Aminopiperidin-1-yl-, 3-(Methylamino)-piperidin-1-yl-, 3-(Ethylamino)-piperidin-1-yl-, 3-(Dimethylamino)-piperidin-1-yl-, 3-(Diethylamino)-piperidin-1-yl-, 3-[(2-Hydroxyethyl)-amino]-piperidin-1-yl-,
3-[N-Methyl-N-(2-hydroxyethyl)-amino]-piperidin-1-yl-, 3-[(3-Hydroxypropyl)amino]-piperidin-1-yl-, 3-[N-Methyl-N-(3-hydroxypropyl)-amino]-piperidin-1-yl-, 3-[(Carboxymethyl)amino]-piperidin-1-yl-, 3-[(Methoxycarbonylmethyl)amino]-piperidin-1-yl-,
3-[(Ethoxycarbonylmethyl)amino]-piperidin-1-yl-, 3-[N-Methyl-N-(methoxycarbonylmethyl)-amino]-piperidin-1-yl-,
3-[N-Methyl-N-(ethoxycarbonylmethyl)-amino]-piperidin-1-yl-, 3-[(2-Carboxyethyl)-amino]-piperidin-1-yl-, 3-{[2-(Methoxycarbonyl)ethyl]amino}-piperidin-1-yl-,
3-{[2-(Ethoxycarbonyl)ethyl]amino}-piperidin-1-yl-, 3-{N-Methyl-N-[2-(methoxycarbonyl)ethyl]-amino}-piperidin-1-yl-, 3-{N-Methyl-N-[2-(ethoxycarbonyl)ethyl]-amino}-piperidin-1-yl-, 3-[(Aminocarbonylmethyl)amino]-piperidin-1-yl-, 3-[(Methylaminocarbonylmethyl)amino]-piperidin-1-yl-, 3-[(Dimethylaminocarbonylmethyl)-amino]-piperidin-1-yl-, 3-[(Ethylaminocarbonylmethyl)amino]-piperidin-1-yl-,
3-[(Diethylaminocarbonylmethyl)amino]-piperidin-1-yl-, 3-[(Pyrrolidin-1-ylcarbonylmethyl)amino]-piperidin-1-yl-, 3-[(2-Cyanpyrrolidin-1-ylcarbonylmethyl)amino]-piperidin-1-yl-,
3-[(4-Cyanthiazolidin-3-ylcarbonylmethyl)amino]-piperidin-1-yl-, 3-[(2-Aminocarbonylpyrrolidin-1-ylcarbonylmethyl)amino]-piperidin-1-yl-, 3-[(2-Carboxypyrrolidin-1-ylcarbonylmethyl)amino]-piperidin-1-yl-, 3-[(2-Methoxycarbonylpyrrolidin-1-ylcarbonylmethyl)amino]-piperidin-1-yl-, 3-[(2-Ethoxycarbonylpyrrolidin-1-ylcarbonylmethyl)-amino]-piperidin-1-yl-, 3-[(Piperidin-1-ylcarbonylmethyl)amino]-piperidin-1-yl-,
3-[(Morpholin-4-ylcarbonylmethyl)amino]-piperidin-1-yl-, 3-Amino-2-methyl-piperidin-1-yl-, 3-Amino-3-methyl-piperidin-1-yl-, 3-Amino-4-methyl-piperidin-1-yl-,
3-Amino-5-methyl-piperidin-1-yl-, 3-Amino-6-methyl-piperidin-1-yl-,
2-Amino-8-aza-bicyclo[3.2.1]oct-8-yl-, 6-Amino-2-aza-bicyclo[2.2.2]oct-2-yl-,
4-Aminopiperidin-1-yl-, 3-Amino-hexahydroazepin-1-yl-, 4-Amino-hexahydroazepin-1-yl-, Piperazin-1-yl-, [1,4]Diazepan-1-yl-, 3-Aminocyclopentyl-, 3-Aminocyclohexyl-, 3-(Methylamino)-cyclohexyl-,
3-(Ethylamino)-cyclohexyl-, 3-(Dimethylamino)-cyclohexyl-, 3-(Diethylamino)-cyclohexyl-, 4-Aminocyclohexyl-, (2-Aminocyclopropyl)amino-, (2-Aminocyclobutyl)amino-, (3-Aminocyclobutyl)amino-, (2-Aminocyclopentyl)amino-, (3-Aminocyclopentyl)amino-, (2-Aminocyclohexyl)amino- oder (3-Aminocyclohexyl)aminogruppe in Betracht.

Bevorzugt sind diejenigen Verbindungen der obigen allgemeinen Formel I, in denen
R¹ eine durch eine Gruppe Rₐ substituierte Methylgruppe, wobei
Rₐ eine 1,4-Dihydro-chinazolinyl- oder 3,4-Dihydro-chinazolinylgruppe,
eine 3,4-Dihydro-isochinollnylgruppe,
eine 1*H*-Benzo[*d*][1,2]oxazinyl- oder 1-Oxo-1H-benzo[*d*][1,2]oxazinylgruppe,
eine 4*H*-Benzo[*e*][1,3]oxazinyl- oder 4-Oxo-4H-benzo[*e*][1,3]oxazinylgruppe,
eine 4*H*-Benzo[*d*][1,3]oxazinyl- oder 4-Oxo-4*H*-benzo[*d*][1,3]oxazinylgruppe,
2*H*-Benzo[1,4]oxazinyl- oder 2-Oxo-2*H-*benzo[1,4]oxazinylgruppe,
eine 4*H*-Benzo[*e*][1,3]thiazinyl- oder 4-Oxo-4*H*-benzo[*e*][1,3]thiazinylgruppe
eine 4*H*-Benzo[*d*][1,3]thiazinyl- oder 2*H*-Benzo[1,4]thiazinylgruppe,
eine 2-Oxo-2*H*-benzo[*e*][1,3]oxazinyl- oder 2,2-Dioxo-1*H-*benzo[*c*][1,2]thiazinylgruppe,
eine 2,3-Dihydro-1*H-*benzo[*e*][1,4]diazepinyl- oder 2-Oxo-2,3-dihydro-1*H-*benzo[*e*][1,4]diazepinylgruppe,
eine 4,5-Dihydro-3*H*-benzo[*b*][1,4]diazepinyl- oder 4-Oxo-4,5-dihydro-3*H-*benzo[*b*][1,4]diazepinylgruppe,
eine 5-Oxo-4,5-dihydro-3*H*-benzo[*e*][1,4]diazepinylgruppe,
eine 2,3-Dihydro-benzo[*f*][1,4]oxazepinyl- oder 2,3-Dihydrobenzo[*b*][1,4]oxazepinylgruppe,
eine 2,3-Dihydro-benzo[*f*][1,4]thiazepinyl- 2,3-Dihydrobenzo[*b*][1,4]thiazepinylgruppe,
eine 5-Oxo-4,5-dihydro-benzo[*f*][1,3,4]oxadiazepinylgruppe,
eine 11*H*-Dibenzo[*b,e*]azepinyl- oder 11-Oxo-11*H*-dibenzo[*b,e*]azepinylgruppe,
eine 11*H-*Benzo[*e*]pyrido[3,2-*b*]azepinylgruppe,
eine 5*H*-Dibenzo[*b,e*][1,4]diazepinyl- oder Dibenzo[*b,f*][1,4]oxazepinylgruppe,
eine Dibenzo[*b,f*][1,4]thiazepinyl-, 5-Oxo-dibenzo[*b,f*][1,4]thiazepinyl- oder 5,5-Dioxo-dibenzo[*b,f*][1,4]thiazepinylgruppe,
5H-Dibenzo[*a,d*]cycloheptenyl- oder 5*H*-Dibenzo[*b,f*]azepinylgruppe,
eine Phenanthridinyl-, Benzo[*c*][1,5]naphthyridinyl-, Benzo[*h*][1,6]naphthyridinyl-, Benzo[*c*][1,8]naphthyridinyl- oder 1,2,3,4-Tetrahydro-phenanthridinylgruppe,
eine Benzo[*f*]chinoxalinylgruppe,
eine 5*H*-Dibenzo[*d*,*f*][1,3]diazepinyl-, 5H-Benzo[*e*]pyrrolo[1,2-a][1,4]diazepinyl- oder Thieno[3,2-*b*][1,4]benzoxazepinylgruppe,
eine 3-Oxo-2,3-dihydro-isoindol-1-ylidengruppe,
eine Benzo[1,2,5]oxadiazolylgruppe,
eine Dibenzofuranylgruppe,
eine Indolizinylgruppe,
eine 1*H-*Perimidinylgruppe,
eine Pyrazolo[1,5-c]chinzolinylgruppe oder
eine Imidazo[2,1-a]isochinolinyl- oder Imidazo[1,2-a]isochinolinylgruppe
bedeutet, wobei die Benzogruppen der vorstehend erwähnten Reste Rₐ durch die Gruppen R¹⁰ bis R¹² substituiert sind und die Alkyleneinheiten der vorstehend erwähnten Reste Rₐ durch eine oder zwei C₁₋₃-Alkyl- oder C₁₋₃-Alkyloxy-carbonylgruppen, wobei die Reste gleich oder verschieden sein können, oder durch eine Trifluormethylgruppe substituiert sein können und die Iminogruppen der vorstehend erwähnten Reste Rₐ durch eine C₁₋₃-Alkylgruppe substituiert sein können und
R¹⁰ ein Wasserstoffatom,
ein Fluor-, Chlor-, Brom- oder lodatom,
eine C₁₋₃-Alkyl- oder Cyclopropylgruppe,
eine Hydroxy-, C₁₋₃-Alkyloxy- oder Cyclopropyloxygruppe,
eine Nitro-, Amino-, C₁₋₃-Alkylamino- oder Di-(C₁₋₃-alkyl)aminogruppe,
eine C₁₋₃-Alkyl-carbonylamino- oder C₁₋₃-Alkyl-sulfonylaminogruppe,
eine Cyan-, Carboxy-, C₁₋₃-Alkyloxy-carbonyl-, Aminocarbonyl-, C₁₋₃-Alkyl-aminocarbonyl- oder Di-(C₁₋₃-alkyl)-aminocarbonylgruppe,
eine Mercapto-, C₁₋₃-Alkylsulfanyl-, C₁₋₃-Alkysulfinyl-,oder C₁₋₃-Alkylsulfonyl- oder Aminosulfonylgruppe oder
eine Difluormethyl-, Trifluormethyl-, Difluormethoxy- oder Trifluormethoxygruppe und
R¹¹ und R¹², die gleich oder verschieden sein können, jeweils ein Wasserstoffatom, ein Fluor-, Chlor- oder Bromatom, eine Methyl-, Trifluormethyl- oder Methoxygruppe bedeuten,
R² ein Wasserstoffatom,
eine C₁₋₃-Alkylgruppe,
eine C₃₋₆Cycloalkylgruppe oder
eine gegebenenfalls durch ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe mono- oder disubstituierte Phenylgruppe, wobei die Substituenten gleich oder verschieden sein können,
R³ eine 2-Buten-1-yl- oder 3-Methyl-2-buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
wobei, soweit nichts anderes erwähnt wurde, die vorstehend erwähnten Alkylgruppen geradkettig oder verzweigt sein könnnen,
deren Tautomere, Enantiomere, Diastereomere, deren Gemische und deren Salze.

Besonders bevorzugt sind diejenigen Verbindungen der obigen allgemeinen Formel 1, in denen
R¹ eine durch eine Gruppe Rₐ substituierte Methylgruppe, wobei
Rₐ eine 1,4-Dihydro-chinazolin-2-yl- oder 3,4-Dihydro-chinazolin-2-ylgruppe,
eine 3,4-Dihydro-isochinolin-1-ylgruppe,
eine 1*H*-Benzo[*d*][1,2]oxazin-4-yl- oder 1-Oxo-1*H*-benzo[*d*][1,2]oxazin-4-ylgruppe,
eine 4*H*-Benzo[*e*][1,3]oxazin-2-yl- oder 4-Oxo-4H-benzo[*e*][1,3]oxazin-2-ylgruppe,
eine 4*H*-Benzo[*d*][1,3]oxazin-2-yl- oder 4-Oxo-4H-benzo[*d*][1,3]oxazin-2-ylgruppe,
2*H*-Benzo[1,4]oxazin-3-yl- oder 2-Oxo-2*H*-benzo[1,4]oxazin-3-ylgruppe,
eine 4*H*-Benzo[*e*][1,3]thiazin-2-yl- oder 4-Oxo-4*H*-benzo[*e*][1,3]thiazin-2-ylgruppe,
eine 4*H*-Benzo[*d*][1,3]thiazin-2-yl- oder 2*H*-Benzo[1,4]thiazin-3-ylgruppe,
eine 2-Oxo-2*H*-benzo[*e*][1,3]oxazin-4-yl- oder 2,2-Dioxo-1*H*-benzo[*c*][1,2]thiazin-4-ylgruppe,
eine 2,3-Dihydro-1*H-*benzo[*e*][1,4]diazepin-5-yl- oder 2-Oxo-2,3-dihydro-1*H-*benzo[*e*][1,4]diazepin-5-ylgruppe,
eine 4,5-Dihydro-3*H*-benzo[*b*][1,4]diazepin-2-yl- oder 4-Oxo-4,5-dihydro-3*H-*benzo[*b*][1,4]diazepin-2-ylgruppe,
eine 5-Oxo-4,5-dihydro-3*H*-benzo[*e*][1,4]diazepin-2-ylgruppe,
eine 2,3-Dihydro-benzo[*f*][1,4]oxazepin-5-yl- oder 2,3-Dihydro-benzo[*b*]-[1,4]oxazepin-4-ylgruppe,
eine 2,3-Dihydro-benzo[*f*][1,4]thiazepin-5-yl- oder 2,3-Dihydro-benzo[*b*]-[1,4]thiazepin-4-ylgruppe,
eine 5-Oxo-4,5-dihydro-benzo[*f*][1,3,4]oxadiazepin-2-ylgruppe,
eine 11*H*-Dibenzo[*b,e*]azepin-6-yl- oder 11-Oxo-11*H-*dibenzo[*b,e*]azepin-6-ylgruppe,
eine 11*H*-Benzo[*e*]pyrido[3,2-*b*]azepin-6-ylgruppe
eine 5*H*-Dibenzo[*b,e*][1,4]diazepin-11-yl- oder Dibenzo[*b,f*][1,4]oxazepin-11-ylgruppe,
eine Dibenzo[*b,f*][1,4]thiazepin-11-yl-, 5-Oxo-dibenzo[*b,f*][1,4]thiazepin-11-yl- oder 5,5-Dioxo-dibenzo[*b,f*][1,4]thiazepin-11-ylgruppe,
eine 5H-Dibenzo[*a,d*]cyclohepten-10-yl- oder 5*H*-Dibenzo[*b,f*]azepin-10-ylgruppe,
eine Phenanthridin-6-yl-, Benzo[*c*][1,5]naphthyridin-6-yl-, Benzo[*h*][1,6]naphthyridin-5-yl-, Benzo[*c*][1,8]naphthyridin-6-yl- oder 1,2,3,4-Tetrahydro-phenanthridin-6-ylgruppe,
eine Benzo[*f*]chinoxalin-6-ylgruppe,
eine 5*H*-Dibenzo[*d,f*][1,3]diazepin-6-yt-, 5H-Benzo[*e*]pyrrolo[1,2-*a*][1,4]diazepin-11-yl- oder Thieno[3,2-b][1,4]benzoxazepinyl-9-ylgruppe,
eine 3-Oxo-2,3-dihydro-isoindol-1-ylidengruppe,
eine Benzo[1,2,5]oxadiazol-5-ylgruppe,
eine Dibenzofuran-2-ylgruppe,
eine Indolizin-2-ylgruppe,
eine 1*H-*Perimidin-2-ylgruppe,
eine Pyrazolo[1,5-c]chinzolin-5-ylgruppe oder
eine Imidazo[2,1-a]isochinolin-2-yl- oder Imidazo[1,2-a]isochinolin-2-ylgruppe
bedeutet, wobei die Benzogruppen der vorstehend erwähnten Reste Rₐ durch die Gruppen R¹⁰ bis R¹² substituiert sind und die Alkyleneinheiten der vorstehend erwähnten Reste Rₐ durch eine oder zwei Methyl- oder Methoxycarbonylgruppen, wobei die Reste gleich oder verschieden sein können, oder durch eine Trifluormethylgruppe substituiert sein können und die Iminogruppen der vorstehend erwähnten Reste Rₐ durch eine Methylgruppe substituiert sein können und
R¹⁰ ein Wasserstoffatom,
ein Fluor-, Chlor-, Brom- oder Iodatom,
eine Methyl- oder Ethylgruppe,
eine Hydroxy-, Methoxy- oder Ethoxygruppe oder
eine Difluormethyl-, Trifluormethyl-, Difluormethoxy-, oder Trifluormethoxygruppe und
R¹¹ und R¹², die gleich oder verschieden sein können, jeweils ein Wasserstoffatom, ein Fluor-, Chlor- oder Bromatom, eine Methyl-, Trifluormethyl- oder Methoxygruppe bedeuten,
R² ein Wasserstoffatom oder
eine Methyl-, Ethyl-, Propyl-, Isopropyl-, Phenyl- oder Cyclopropylgruppe,
R³ eine 2-Buten-1-yl- oder 3-Methyl-2-buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
deren Tautomere, Enantiomere, Diastereomere, deren Gemische und deren Salze.

Ganz besonders bevorzugt sind diejenigen Verbindungen der obigen allgemeinen Formel I, in denen
R¹ eine 3-Methoxycarbonyl-3-methyl-3,4-dihydro-isochinolin-1-ylmethylgruppe,
eine 1-Methyl-2,2-dioxo-1*H-*benzo[*c*][1,2]thiazin-4-ylmethylgruppe,
eine 2,3-Dihydro-benzo[*f*][1,4]oxazepin-5-ylmethylgruppe,
eine 2-Oxo-2,3-dihydro-1*H*-benzo[*e*][1,4]diazepin-5-ylmethylgruppe,
eine Phenanthridin-6-ylmethyl- oder 1,2,3,4-Tetrahydro-phenanthridin-6-ylmethylgruppe,
eine 11*H*-Dibenzo[*b*,*e*]azepin-6-ylmethylgruppe,
eine Dibenzo[*b,f*][1,4]oxazepin-11-ylmethylgruppe,
eine 3-Oxo-2,3-dihydro-isoindol-1-ylidenmethylgruppe,
eine 3-Trifluormethyl-3,4-dihydro-isochinolin-1-ylmethylgruppe,
eine 3,4-Dihydro-chinazolin-2-ylmethylgruppe,
eine 5-Methyl-5*H*-dibenzo[*b,e*][1,4]diazepin-11-ylmethylgruppe,
eine 8-Methyl-dibenzo[*b,f*][1,4]oxazepin-11-ylmethylgruppe,
eine Benzo[1,2,5]oxadiazol-5-ylmethylgruppe,
eine 8-Methyl-phenanthridin-6-ylmethylgruppe,
eine 1-Methyl-phenanthridin-6-ylmethylgruppe,
eine 4-Methyl-phenanthridin-6-ylmethylgruppe,
eine Benzo[*h*][1,6]naphthyridin-5-ylmethylgruppe,
eine Pyrazolo[1,5-c]chinzolin-5-ylgruppe,
eine Benzo[*c*][1,8]naphthyridin-6-ylmethylgruppe,
eine Benzo[*c*][1,5]naphthyridin-6-ylmethylgruppe,
eine 1*H*-Perimidin-2-ylmethylgruppe,
eine Benzo[*f*]chinoxalin-6-ylmethylgruppe oder
eine Imidazo[2,1-a]isochinolin-2-ylmethyl- oder Imidazo[1,2-a]isochinolin-2-ylmethylgruppe,
R² eine Methyl- oder Cyclopropylgruppe,
R³ eine 2-Buten-1-yl-, 3-Methyl-2-buten-1-yl- oder 2-Butin-1-ylgruppe
und
R⁴ eine (3-Amino-piperidin-1-yl)gruppe bedeuten, deren Tautomere, Enantiomere, Diastereomere, deren Gemische und deren Salze.

Folgende Verbindungen der allgemeinen Formel I sind besonders bevorzugt:
(1) 1-[(1-Methyl-2,2-dioxo-1*H*-benzo[*c*][1,2]thiazin-4-yl)methyl]-3-methyl-7-(3-methyl-2-buten-1-yl)-8-(3-amino-piperidin-1-yl)-xanthin,
(2) 1-[(3-Methoxycarbonyl-3-methyl-3,4-dihydro-isochinolin-1-yl]methyl]-3-methyl-7-(2-butin-1-yl)-8-(3-amino-piperidin-1-yl)-xanthin,
(3) 1-[(2-Oxo-2,3-dihydro-1H-benzo[*e*][1,4]diazepin-5-yl)methyl]-3-methyl-7-((E)-2-buten-1-yl)-8-((*R*)-3-amino-piperidin-1-yl)-xanthin,
(4) 1-[(Phenanthridin-6-yl)methyl]-3-methyl-7-(2-butin-1-yl)-8-(3-amino-piperidin-1-yl)-xanthin,
(5) 1-[(1,2,3,4-Tetrahydro-phenanthridin-6-yl)methyl]-3-methyl-7-(2-butin-1-yl)-8-(3-amino-piperidin-1-yl)-xanthin,
(6) 1-[(11*H*-Dibenzo[*b,e*]azepin-6-yl)methyl]-3-methyl-7-(2-butin-1-yl)-8-(3-aminopiperidin-1-yl)-xanthin,
(7) 1-[(Dibenzo[*b,f*][1,4]oxazepin-11-yl)methyl]-3-methyl-7-(2-butin-1-yl)-8-(3-aminopiperidin-1-yl)-xanthin,
(8) 1-[(3-Trifluormethyl-3,4-dihydro-isochinolin-1-yl)methyl]-3-methyl-7-(2-butin-1-yl)-8-(3-amino-piperidin-1-yl)-xanthin,
(9) 1-[(Dibenzo[*b,f*][1,4]oxazepin-11-yl)methyl]-3-methyl-7-(2-butin-1-yl)-8-((*R*)-3-amino-piperidin-1-yl)-xanthin,
(10) 1-[(3,4-Dihydro-chinazolin-2-yl)methyl]-3-methyl-7-(2-butin-1-yl)-8-(3-aminopiperidin-1-yl)-xanthin,
(11) 1-[(5-Methyl-5*H*-dibenzo[*b,e*][1,4]diazepin-11-yl)methyl]-3-methyl-7-(2-butin-1-yl)-8-(3-amino-piperidin-1-yl)-xanthin,
(12) 1-[(8-Methyl-dibenzo[*b,f*][1,4]oxazepin-11-yl)methyl]-3-methyl-7-(2-butin-1-yl)-8-(3-amino-piperidin-1-yl)-xanthin,
(13) 1-[(Benzo[1,2,5]oxadiazol-5-yl)methyl]-3-methyl-7-(2-butin-1-yl)-8-(3-aminopiperidin-1-yl)-xanthin,
(14) 1-[(Phenanthridin-6-yl)methyl]-3-cyclopropyl-7-(2-butin-1-yl)-8-(3-aminopiperidin-1-yl)-xanthin,
(15) 1-[(8-Methyl-phenanthridin-6-yl)methyl]-3-methyl-7-(2-butin-1-yl)-8-(3-aminopiperidin-1-yl)-xanthin,
(16) 1-[(Phenanthridin-6-yl)methyl]-3-methyl-7-(2-butin-1-yl)-8-((*S*)-3-amino-piperidin-1-yl)-xanthin ,
(17) 1-[(Phenanthridin-6-yl)methyl]-3-cyclopropyl-7-(2-butin-1-yl)-8-((*R*)-3-aminopiperidin-1-yl)-xanthin,
(18) 1-[(1-Methy)-phenanthridin-6-yl)methyl]-3-methyl-7-(2-butin-1-yl)-8-(*R*)-3-aminopiperidin-1-yl)-xanthin,
(19) 1-[(4-Methyl-phenanthridin-6-yl)methyl]-3-methyl-7-(2-butin-1-yl)-8-((*R*)-3-aminopiperidin-1-yl)-xanthin,
(20) 1-[(Benzo[*h*][1,6]naphthyridin-5-yl)methyl]-3-methyl-7-(2-butin-1-yl)-8-(3-aminopiperidin-1-yl)-xanthin,
(21) 1-[(Pyrazolo[1,5-c]chinazolin-5-yl)methyl]-3-methyl-7-(2-butin-1-yl)-8-(3-aminopiperidin-1-yl)-xanthin,
(22) 1-[(Benzo[*c*][1,8]naphthyridin-6-yl)methyl]-3-methyl-7-(2-butin-1-yl)-8-(3-aminopiperidin-1-yl)-xanthin,
(23) 1-[(Benzo[*c*][1,5]naphthyridin-6-yl)methyl]-3-methyl-7-(2-butin-1-yl)-8-((*R*)-3-amino-piperidin-1-yl)-xanthin,
(24) 1-[(1*H*-Perimidin-2-yl)methyl]-3-methyl-7-(2-butin-1-yl)-8-((*R*)-3-amino-piperidin-1-yl)-xanthin,
(25) 1-[(Benzo[*f*]chihoxalin-6-yl)methyl]-3-methyl-7-(2-butin-1-yl)-8-((*R*)-3-aminopiperidin-1-yl)-xanthin,
(26) 1-[(Imidazo[2,1-a]isochinolin-2-yl)methyl]-3-methyl-7-(2-butin-1-yl)-8-(3-aminopiperidin-1-yl)-xanthin,
(27) 1-[(Imidazo[1,2-a]isochinolin-2-yl)methyl]-3-methyl-7-(2-butin-1-yl)-8-(3-aminopiperidin-1-yl)-xanthin,
(28) 1-[(Phenanthridin-6-yl)methyl]-3-methyl-7-(2-butin-1-yl)-8-((*R*)-3-amino-piperidin-1-yl)-xanthin,
(29) 1-[(2,3-Dihydro-benzo[*f*][1,4]oxazepin-5-yl)methyl]-3-methyl-7-(2-butin-1-yl)-8-(3-amino-piperidin-1-yl)-xanthin, und
(30) 1-[(3-Oxo-2,3-dihydro-isoindol-1-yliden)methyl]-3-methyl-7-(3-methyl-2-buten-1-yl)-8-(3-amino-piperidin-1-yl)-xanthin
sowie deren Salze.

Erfindungsgemäß erhält man die Verbindungen der allgemeinen Formel I nach an sich bekannten Verfahren, beispielsweise nach folgenden Verfahren:
a) Entschützung einer Verbindung der allgemeinen Formel 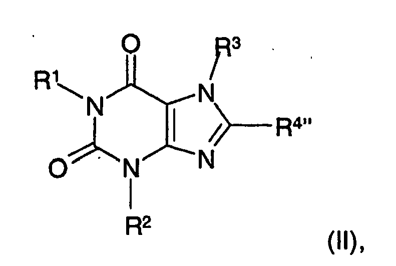 in der R¹, R² und R³ wie eingangs erwähnt definiert sind und
   R^{4'}' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe substituiert ist.
   Die Freisetzung einer Aminogruppe aus einer geschützten Vorstufe ist eine Standardreaktion in der synthetischen organischen Chemie. Als Schutzgruppen kommen eine Vielzahl von Gruppen in Frage. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocienski, Protecting Groups, Georg Thieme Verlag, 1994.
   Als Beispiele für Schutzgruppen seien genannt:
   die tert.-Butyloxycarbonylgruppe, die sich durch Behandeln mit einer Säure wie beispielsweise Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C abspalten lässt,
   die 2.2.2-Trichlorethoxycarbonylgruppe, die sich abspalten lässt durch Behandeln mit Metallen wie beispielsweise Zink oder Cadmium in einem Lösungsmittel wie Essigsäure oder einem Gemisch aus Tetrahydrofuran und einer schwachen wässrigen Säure bei Temperaturen zwischen 0°C und der Siedetemperatur des verwendeten Lösungsmittels und
   die Carbobenzyloxycarbonylgruppe, die sich beispielsweise abspalten lässt durch Hydrogenolyse in Gegenwart eines Edelmetallkatalysators wie beispielsweise Palladium-Kohle und einem Lösungsmittel wie beispielsweise Alkohole, Essigester, Dioxan, Tetrahydrofuran oder Gemische dieser Lösungsmittel bei Temperaturen zwischen 0°C und dem Siedepunkt des Lösungsmittels, durch Behandeln mit Bortribromid in Methylenchlorid bei Temperaturen zwischen -20°C und Raumtemperatur, oder durch Behandeln mit Aluminiumchlorid/Anisol bei Temperaturen zwischen 0°C und Raumtemperatur.
b) Entschützung und Cyclisierung einer Verbindung der allgemeinen Formel 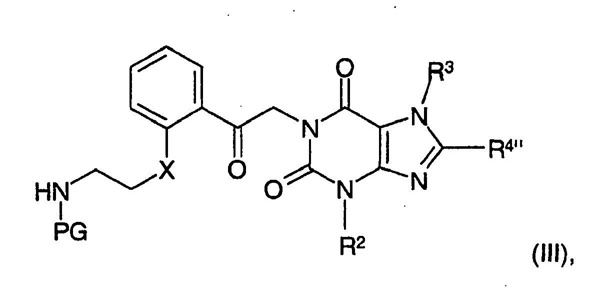 in der R² und R³ wie eingangs erwähnt definiert sind, R^{4'}' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine der vorstehend erwähnten Schutzgruppe substituiert ist, X ein Sauerstoff- oder Schwefelatom, eine Sulfinyl-, Sulfonyl- oder eine durch Rₓ substituierte Iminogruppe bedeutet, und
   die -CH₂-CH₂-X-Phenyl-Einheit durch R¹⁰ bis R¹⁴ substituiert ist und zusätzlich durch eine C₁₋₃-Alkylgruppe substituiert sein kann, wobei Rₓ und R¹⁰ bis R¹⁴ wie eingangs erwähnt definiert sind, und PG ebenfalls eine der vorstehend erwähnten Schutzgruppen bedeutet, wobei beide Schutzgruppen gleichzeitig oder nacheinander abspaltbar sind (siehe Beispiel 2).
c) Zur Herstellung einer Verbindung der allgemeinen Formel I, in der R¹ eine gegebenenfalls durch eine in den Ansprüchen 1 bis 4 definierte Gruppe substituierte 3-Oxo-2,3-dihydro-isoindol-1-ylidenmethylgruppe darstellt:
   Entschützung und Dehydratisierung einer Verbindung der allgemeinen Formel 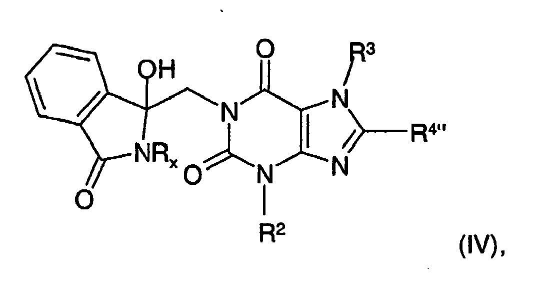 in der die Benzogruppe durch R¹⁰ bis R¹⁴ substituiert ist,
   und R¹⁰ bis R¹⁴ sowie Rₓ, R² und R³ und wie eingangs erwähnt definiert sind, und R^{4'}' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine der vorstehend erwähnten Schutzgruppe substituiert ist und die Dehydratisierung unter den gleichen Reaktionsbedingungen wie die Abspaltung der Schutzgruppe erfolgt (siehe Beispiel 3).

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II, III und IV sind entweder literaturbekannt oder man erhält diese nach an sich literaturbekannten Verfahren (siehe Beispiele I bis XV).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:
Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag.

Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 1 | 13 |
| 1(1) | 32 |
| 1(2) | 6 |
| 1(3) | 5 |
| 1(4) | 5 |
| 1(7) | 11 |
| 1(8) | 4 |
| 1(10) | 8 |
| 1(12) | 14 |
| 1(15) | 11 |
| 1(20) | 10 |
| 1(25) | 7 |
| 1(26) | 7 |
| 1(27) | 2 |
| 1(28) | 2 |
| 1(29) | 3 |
| 1(30) | 3 |
| 1(32) | 4 |
| 1(33) | 4 |
| 2 | 6 |
| 3 | 20 |

Die erfindungsgemäß hergestellten Verbindungen sind gut verträglich, da beispielsweise nach oraler Gabe von 10 mg/kg der Verbindung des Beispiels 1 (2) an Ratten keine Änderungen im Verhalten der Tiere beobachtet werden konnten

Im Hinblick auf die Fähigkeit, die DPP-IV Aktivität zu hemmen, sind die erfindungsgemäßen Verbindungen der allgemeinen Formel I und ihre entsprechenden, pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ I und Typ II, diabetische Komplikationen, metabolische Azidose oder Ketose, Insulinresistenz, Dyslipidämien unterschiedlichster. Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Ebenso sind sie zur Prävention und Therapie von chronischen entzündlichen Darmerkrankungen geeignet. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Des weiteren sind die Substanzen geeignet, Mangelzustände von Wachstumshormon, die mit Minderwuchs einhergehen, zu beeinflussen.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570), alpha-Glucosidasehemmer (z.B. Acarbose, Voglibose), alpha2-Antagonisten, Insulin und Insulinanaloga, GLP-1 und GLP-1 Analoga (z.B. Exendin-4) oder Amylin. Daneben Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, Cholesterolresorptionsinhibitoren wie zum Beispiel Ezetimibe, gallensäurebindende Substanzen wie zum Beispiel Colestyramin, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin oder β3-Agonisten wie SB-418790 oder AD-9677.

Daneben ist eine Kombination mit Medikamenten zur Beeinflussung des Bluthochdrucks wie z.B. All Antagonisten oder ACE Inhibitoren, Diuretika, β-Blocker und andere oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder VerdünnungsMitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:
Herstellung der Ausgangsverbindungen:

### Beispiel I

### 1-[(1-Methyl-2,2-dioxo-1H-benzo[c][1,2]thiazin-4-yl)methyl]-3-methyl-7-(3-methyl-2-buten-1-yl)-8-[3-(tert.-butyloxycarbonylamino)-piperidin-1-yl]-xanthin

Ein Gemisch aus 260 mg 3-Methyl-7-(3-methyl-2-buten-1-yl)-8-[3-(tert.-butyloxycarbonylamino)-piperidin-1-yl]-xanthin, 185 mg 4-Brom-methyl-1-methyl-1*H-*benzo[*c*][1,2]thiazin-2,2-dioxid und 550 mg Kaliumcarbonat in 4 ml N,N-Dimethylformamid wird etwa 40 h bei Raumtemperatur gerührt. Da laut Dünnschichtchromatogramm keine nennenswerte Umsetzung erkennbar ist, wird das Gemisch für 2 h auf 60° C erhitzt und anschließend noch 15 h bei 50°C gerührt, bis die Umsetzung nahezu vollständig ist. Dann werden 30 ml Wasser zugegeben, der entstandene Niederschlag wird abgesaugt und getrocknet. Das Rohprodukt wird chromatographisch über eine Kieselgelsäule mit Petrolether/Essigester (1:1) als Laufmittel gereinigt.
Ausbeute: 225 mg (59 % der Theorie)
R_{f}-Wert: 0.19 (Kieselgel, Petrolether/Essigester = 1:1)
Massenspektrum (ESI⁺): m/z = 640 [M+H]⁺

Analog Beispiel I werden folgende Verbindungen erhalten:
(1) 1-[(3-Methoxycarbonyl-3-methyl-3,4-dihydro-isochinolin-1-yl]methyl]-3-methyl-7-(2-butin-1-yl)-8-[3-(tert.-butyloxycarbonylamino)-piperidin-1-yl]-xanthin R_{f}-Wert: 0.42 (Kieselgel, Methylenchlorid/Methanoi/konz. wässriges Ammoniak = 95:5)
   Massenspektrum (ESI⁺): m/z = 632 [M+H]⁺
(2) 1-[2-(2-Methoxy-phenyl)-2-oxo-ethyl]-3-methyl-7-(2-butin-1-yl)-8-brom-xanthin Massenspektrum (ESI⁺): m/z = 445, 447 [M+H]⁺
(3) 1-[2-(2-Ethoxycarbonyl-phenyl)-2-oxo-ethyl]-3-methyl-7-(3-methyl-2-buten-1-yl)-8-[3-(tert.-buty)oxycarbonylamino)-piperidin-1-yl]-xanthin
   (Durchführung in N-Methylpyrrolidin-2-on bei 60°C)
   R_{f}-Wert: 0.35 (Kieselgel, Methylenchlorid/Methanol = 20:1)
   Massenspektrum (ESI⁺): m/z = 623 [M+H]⁺
(4) 1-[2-(2-Nitro-phenyl)-2-oxo-ethyl]-3-methyl-7-((E)-2-buten-1-yl)-8-brom-xanthin Massenspektrum (ESI⁺): m/z = 462, 464 [M+H]⁺
(5) 1-[(Phenanthridin-6-yl)methyl]-3-methyl-7-(2-butin-1-yl)-8-[3-(tert.-butyloxycarbonylamino)-piperidin-1-yl]-xanthin
   R_{f}-Wert: 0.80 (Kieselgel, Essigester)
   Massenspektrum (ESI⁺): m/z = 608 [M+H]⁺
(6) 1-[(1,2,3,4-Tetrahydro-phenanthridin-6-yl)methyl]-3-methyl-7-(2-butin-1-yl)-8-[3-(tert.-butyloxycarbonylamino)-piperidin-1-yl]-xanthin
   R_{f}-Wert: 0.55 (Kieselgel, Essigester/ Petrolether = 2:1)
   Massenspektrum (ESI⁺): m/z = 612 [M+H]⁺
(7) 1-[(11*H*-Dibenzo[*b*,*e*]azepin-6-yl)methyl]-3-methyl-7-(2-butin-1-yl)-8-[3-(tert.-butyloxycarbonylamino)-piperidin-1-yl]-xanthin
   R_{f}-Wert: 0.40 (Kieselgel, Cyclohexan/Essigester = 1:1)
   Massenspektrum (ESI⁺): m/z = 622 [M+H]⁺
(8) 1-[(Dibenzo[*b*,*f*][1,4]oxazepin-11-yl)methyl]-3-methyl-7-(2-butin-1-yl)-8-[3-(tert.-butyloxycarbonylamino)-piperidin-1-yl]-xanthin
   R_{f}-Wert: 0.70 (Kieselgel, Essigester/Cyclohexan = 3:1)
   Massenspektrum (ESI⁺): m/z = 624 [M+H]⁺
(9) 1-[(3-Trifluormethyl-3,4-dihydro-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.60 (Aluminiumoxid, Petrolether/Essigester = 1:1)
   Massenspektrum (ESI⁺): m/z = 628 [M+H]⁺
(10) 1-[(Dibenzo[*b*,*f*][1,4]oxazepin-11-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Essigester)
   Massenspektrum (ESI⁺): m/z = 624 [M+H]⁺
(11) 1-[(3,3-Dimethyl-3,4-dihydro-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Essigester)
   Massenspektrum (ESI⁺): m/z = 588 [M+H]⁺
(12) 1-[(Methoxycarbonyl)methyl]-3-methyl-7-(2-butin-1-yl)-8-[3-(tert.-butyloxycarbonyl-amino)-piperidin-1-yl]-xanthin
   Massenspektrum (ESI⁺): m/z = 489 [M+H]⁺
(13) 1-Cyanomethyl-3-methyl-7-(2-butin-1-yl)-8-[3-(tert.-butyloxycarbonyl-amino)-piperidin-1-yl]-xanthin
   Massenspektrum (ESI⁺): m/z = 456 [M+H]⁺
(14) 1-[(5-Methyl-5*H*-dibenzo[*b*,*e*][1,4]diazepin-11-yl)methyl]-3-methyl-7-(2-butin-1-yl)-8-[3-(tert.-butyloxycarbonyl-amino)-piperidin-1-yl]-xanthin
   R_{f}-Wert: 0.60 (Kieselgel, Methylenchlorid/Essigester = 1:1)
   Massenspektrum (ESI⁺): m/z = 637 [M+H]⁺
(15) 1-[(8-Methyl-dibenzo[*b*,*f*][1,4]oxazepin-11-yl)methyl]-3-methyl-7-(2-butin-1-yl)-8-[3-(tert.-butyloxycarbonyl-amino)-piperidin-1-yl]-xanthin
   R_{f}-Wert: 0.65 (Kieselgel, Methylenchlorid/Essigester= 1:1)
   Massenspektrum (ESI⁺): m/z = 638 [M+H]⁺
(16) 1-[(2-Methyl-dibenzo[*b*,*f*][1,4]oxazepin-11-yl)methyl]-3-methyl-7-(2-butin-1-yl)-8-[3-(tert.-butyloxycarbonyl-amino)-piperidin-1-yl]-xanthin
   R_{f}-Wert: 0.70 (Kieselgel, Methylenchlorid/Essigester = 1:1)
   Massenspektrum (ESI⁺): m/z = 638 [M+H]⁺
(17) 1-[(Benzo[1,2,5]oxadiazol-5-yl)methyl]-3-methyl-7-(2-butin-1-yl)-8-[3-(tert.-butyloxycarbonyl-amino)-piperidin-1-yl]-xanthin
   R_{f}-Wert: 0.73 (Kieselgel, Methylenchlorid/Essigester = 1:1)
   Massenspektrum (ESI⁺): m/z = 549 [M+H]⁺
(18) 1-[(2-Chlor-dibenzo[*b*,*f*][1,4]oxazepin-11-yl)methyl]-3-methyl-7-(2-butin-1-yl)-8-[3-(tert.-butyloxycarbonyl-amino)-piperdin-1-yl]-xanthin
   R_{f}-Wert: 0.75 (Kieselgel, Methylenchlorid/Essigester = 1:1)
   Massenspektrum (ESI⁺): m/z = 658, 660 [M+H]⁺
(19) 1-[(Phenanthridin-6-yl)methyl]-3-cyclopropyl-7-(2-butin-1-yl)-8-[3-(tert.-butyloxycarbonyl-amino)-piperidin-1-yl]-xanthin
   R_{f}-Wert: 0.55 (Kieselgel, Methylenchlorid/Essigester = 1:1)
   Massenspektrum (ESI⁺): m/z = 634 [M+H]⁺
(20) 1-[(8-Methyl-phenanthridin-6-yl)methyl]-3-methyl-7-(2-butin-1-yl)-8-[3-(tert.-butyloxycarbonyl-amino)-piperidin-1-yl]-xanthin
   R_{f}-Wert: 0.67 (Kieselgel, Methylenchlorid/Essigester = 1:1)
   Massenspektrum (ESI⁺): m/z = 622 [M+H]⁺
(21) 1-[(Phenanthridin-6-yl)methyl]-3-methyl-7-(2-butin-1-yl)-8-[(*S*)-3-(tert.-butyloxycarbonyl-amino)-piperidin-1-yl]-xanthin
   R_{f}-Wert: 0.75 (Kieselgel, Methylenchlorid/Essigester = 1:1)
   Massenspektrum (ESI⁺): m/z = 608 [M+H]⁺
(22) 1-[(Phenanthridin-6-yl)methyl]-3-cyclopropyl-7-(2-butin-1-yl)-8-[(*R*)-3-(tert.-butyloxycarbonyl-amino)-piperidin-1-yl]-xanthin
   R_{f}-Wert: 0.60 (Kieselgel, Methylenchlorid/Essigester = 1:1)
   Massenspektrum (ESI⁺): m/z = 634 [M+H]⁺
(23) 1-[(Dibenzofuran-2-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.85 (Kieselgel, Methylenchlorid/Essigester = 1:1)
   Massenspektrum (ESI⁺): m/z = 597 [M+H]⁺
(24) 1-[(1-Methyl-phenanthridin-6-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.80 (Kieselgel, Methylenchlorid/Essigester = 1:1)
   Massenspektrum (ESI⁺): m/z = 622 [M+H]⁺
(25) 1-[(4-Methyl-phenanthridin-6-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.85 (Kieselgel, Methylenchlorid/Essigester = 1:1)
   Massenspektrum (ESI⁺): m/z = 622 [M+H]⁺
(26) 1-[(Benzo[*h*][1,6]naphthyridin-5-yl)methyl]-3-methyl-7-(2-butin-1-yl)-8-[3-(tert.-butyloxycarbonyl-amino)-piperidin-1-yl]-xanthin
   R_{f}-Wert: 0.54 (Kieselgel, Methylenchlorid/Methanol = 94:6)
   Massenspektrum (ESI⁺): m/z = 609 [M+H]⁺
(27) 1-[(Pyrazolo[1,5-c]chinazolin-5-yl)methyl]-3-methyl-7-(2-butin-1-yl)-8-[3-(tert.-butyloxycarbonyl-amino)-piperidin-1-yl]-xanthin
   R_{f}-Wert: 0.67 (Kieselgel, Essigester)
   Massenspektrum (ESI⁺): m/z = 598 [M+H]⁺
(28) 1-[(Benzo[c][1,8]naphthyridin-6-yl)methyl]-3-methyl-7-(2-butin-1-yl)-8-[3-(tert.-butyloxycarbonyl-amino)-piperidin-1 -yl]-xanthin
   R_{f}-Wert: 0.40 (Kieselgel, Methylenchlorid/Methanol = 95:5)
   Massenspektrum (ESI⁺): m/z = 609 [M+H]⁺
(29) 1-[(Benzo[c][1,5]naphthyridin-6-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.55 (Kieselgel, Methylenchlorid/Methanol = 95:5)
   Massenspektrum (ESI⁺): m/z = 609 [M+H]⁺
(30) 1-Cyanomethyl-3-methyl-7-(2-butin-1-yl)-8-[(*R*)-3-(tert.-butyloxycarbonyl-amino)-piperidin-1-yl]-xanthin
   R_{f}-Wert: 0.80 (Kieselgel, Essigester)
   Massenspektrum (ESI⁺): m/z = 456 [M+H]⁺
(31) 1-[(Benzo[*f*]chinoxalin-6-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.48 (Kieselgel, Methylenchlorid/Methanol = 95:5)
   Massenspektrum (ESI⁺): m/z = 609 [M+H]⁺
(32) 1-[(Imidazo[2,1-a]isochinolin-2-yl)methyl]-3-methyl-7-(2-butin-1-yl)-8-[3-(tert.-butyloxycarbonyl-amino)-piperidin-1-yl]-xanthin
   R_{f}-Wert: 0.47 (Kieselgel, Methylenchiorid/Methano) = 95:5)
   Massenspektrum (ESI⁺): m/z = 597 [M+H]⁺
(33) 1-[(Imidazo[1,2-a]isochinolin-2-yl)methyl]-3-methyl-7-(2-butin-1-yl)-8-[3-(tert.-butyloxycarbonyl-amino)-piperidin-1-yl]-xanthin
   R_{f}-Wert: 0.14 (Kieselgel, Essigester)
   Massenspektrum (ESI⁺): m/z = 597 [M+H]⁺
(34) 1-[(Phenanthridin-6-yl)methyl]-3-methyl-7-(2-butin-1-yl)-8-[(*R*)-3-(tert.-butyloxycarbonyl-amino)-piperidin-1-yl]-xanthin
   R_{f}-Wert: 0.20 (Kieselgel, Cyclohexan/Essigester = 1:1)
   Massenspektrum (ESI⁺): m/z = 608 [M+H]⁺

### Beispiel II

### 4-Brom-methyl-1-methyl-1H-benzo[c][1.2]thiazin-2,2-dioxid

390 mg 1,4-Dimethyl-1*H*-benzo[*c*][1,2]thiazin-2,2-dioxid in 20 ml 1,2-Dichlorethan werden mit 332 mg N-Bromsuccinimid und 50 mg 2,2'-Azodiisobutyronitril versetzt. Die gelbe Lösung wird insgesamt 10 h unter Rückfluß erhitzt und anschließend noch zwei Tage bei Raumtemperatur stehengelassen. Das Reaktionsgemisch wird zwischen Wasser und Methylenchlorid verteilt, die organische Phase mit Wasser nachgewaschen, über Magnesiumsulfat getrocknet und eingeengt. Es bleibt ein gelbliches Harz zurück, welches über eine Kieselgelsäule mit Petrolether/Essigester (5:1 auf 4:1) als Laufmittel gereinigt wird. Es wird ein Gemisch aus 4-Brom-methyl-1-methyl-1*H*-benzo[*c*][1,2]thiazin-2,2-dioxid und 3-Brom-1,4-dimethyl-1*H*-benzo[*c*][1, 2]thiazin-2,2-dioxid erhalten, welches als solches weiter umgesetzt wird.
Aubeute: 190 mg (35 % der Theorie)
Massenspektrum (ESI⁺): m/z = 288, 290 [M+H]⁺

Analog Beispiel II werden folgende Verbindungen erhalten:
(1) 5-Brommethyl-benzo[*h*][1,6]naphthyridin
   R_{f}-Wert: 0.76 (Kieselgel, Essigester/Petrolether = 1:1)
   Massenspektrum (ESI⁺): m/z = 273, 275 [M+H]⁺
(2) 6-Chlormethyl-benzo[*c*][1,8]naphthyridin
   (Durchführung mit N-Chlorsuccinimid in Gegenwart von Benzoylperoxid in Tetrachlorkohlenstoff)
   R_{f}-Wert: 0.47 (Kieselgel, Essigester/Methanol = 98:2)
(3) 6-Brommethyl-benzo[*c*][1,5]naphthyridin
   (Durchführung in Gegenwart von Benzoylperoxid in Tetrachlorkohlenstoff)
   R_{f}-Wert: 0.64 (Kieselgel, Essigester/Petrolether = 1:2)
   Massenspektrum (ESI⁺): m/z = 273, 275 [M+H]⁺
(4) 6-Brommethyl-benzo[*f*]chinoxalin
   (Durchführung in Gegenwart von Benzoylperoxid in Tetrachlorkohlenstoff)
   R_{f}-Wert: 0.33 (Kieselgel, Essigester/Petrolether = 1:5)
   Massenspektrum (ESI⁺): m/z = 273, 275 [M+H]⁺

### Beispiel III

### 3-Methyl-7-(3-methyl-2-buten-1-yl)-8-[3-(tert.-butyloxycarbonylamino)-piperidin-1-yl]-xanthin

Ein Gemisch aus 20.50 g 3-Methyl-7-(3-methyl-2-buten-1-yl)-8-brom-xanthin, 13.64 g 3-tert.-Butyloxycarbonylamino-piperidin und 20.27 g Kaliumcarbonat in 100 ml Dimtheylsulfoxid wird 4 h bei 115°C gerührt. Dann werden nochmals 2.50 g 3-tert.-Butyloxycarbonylamino-piperidin zugegeben und das Reaktionsgemisch wird weitere 2 h bei 115°C gerührt. Die abgekühlte Reaktionslösung wird auf 1 I Eiswasser gegossen, der entstandene Niederschlag abgesaugt, mit Wasser nachgewaschen und getrocknet.
R_{f}-Wert: 0.60 (Kieselgel, Essigester)
Massenspektrum (ESI⁺): m/z = 433 [M+H]⁺

Analog Beispiel III werden folgende Verbindungen erhalten:
(1) 3-Methyl-7-(2-butin-1-yl)-8-[3-(tert.-butyloxycarbonylamino)-piperidin-1-yl]-xanthin Schmelzpunkt: 235-237°C
   Massenspektrum (ESI⁺): m/z = 417 [M+H]⁺
(2) 1-[2-(2-Hydroxy-phenyl)-2-oxo-ethyl]-3-methyl-7-(2-butin-1-yl)-8-[3-(tert.-butyloxycarbonyl-amino)-piperidin-1-yl]-xanthin
   Massenspektrum (ESI⁺): m/z = 551 [M+H]⁺
(3) 1-[2-(2-Nitro-phenyl)-2-oxo-ethyl]-3-methyl-7-((E)-2-buten-1-yl)-8-[(*R*)-3-(tert.-butyloxycarbonylamino)-piperidin-1-yl]-xanthin
   R_{f}-Wert: 0.50 (Kieselgel, Cyclohexan/Essigester = 1:2)
   Massenspektrum (ESI⁺): m/z = 582 [M+H]⁺
(4) 3-Methyl-7-(2-butin-1-yl)-8-[(*R*)-3-(tert.-butyloxycarbonylamino)-piperidin-1-yl]-xanthin
   Massenspektrum (ESI⁺): m/z = 417 [M+H]⁺
(5) 3-Cyclopropyl-7-(2-butin-1-yl)-8-[3-(tert.-butyloxycarbonyl-amino)-piperidin-1-yl]-xanthin
   R_{f}-Wert: 0.70 (Kieselgel, Essigester)
   Massenspektrum (ESI⁺): m/z = 443 [M+H]⁺
(6) 3-Methyl-7-(2-butin-1-yl)-8-[(*S*)-3-(tert.-butyloxycarbonyl-amino)-piperidin-1-yl]-xanthin
   R_{f}-Wert: 0.73 (Kieselgel, Essigester)
   Massenspektrum (ESI⁺): m/z = 417 [M+H]⁺
(7) 3-Cyclopropyl-7-(2-butin-1-yl)-8-[(*R*)-3-(tert.-butyloxycarbonyl-amino)-piperidin-1-yl]-xanthin
   R_{f}-Wert: 0.35 (Reversed Phase DC-Fertigplatte (E. Merck), Acetonitril/Wasser/Trifluoressigsäure = 50:50:1)
   Massenspektrum (ESI⁺): m/z = 443 [M+H]⁺
(8) 1-[(lndolizirl-2-yl)methyl]-3-methyl-7-(2-butin-1-yl)-8-[3-(tert.-butyloxycarbonylamino)-piperidin-1-yl]-xanthin
   R_{f}-Wert: 0.29 (Kieselgel, Petrolether/Essigester = 1:1)
   Massenspektrum (ESI⁺): m/z = 546 [M+H]⁺

### Beispiel IV

### 3-Methyl-7-(3-methyl-2-buten-1-yl)-8-brom-xanthin

Zu 20.00 g 3-Methyl-8-brom-xanthin in 200 ml N,N-Dimethylformamid werden 15.37 ml Hünigbase und 9.98 ml 3,3-Dimethylallylbromid gegeben. Das Reaktionsgemisch wird etwa eine halbe Stunde bei Raumtemperatur gerührt und anschließend mit 500 ml Wasser verdünnt. Der entstandene Niederschlag wird abgesaugt, mit Wasser nachgewaschen und getrocknet.
Ausbeute: 20.50 g (80 % der Theorie)
Massenspektrum (ESI⁺): m/z = 313, 315 [M+H]⁺

Analog Beispiel IV werden folgende Verbindungen erhalten:
(1) 3-Methyl-7-(2-butin-1-yl)-8-brom-xanthin
   R_{f}-Wert: 0.72 (Kieselgel, Essigester)
   Massenspektrum (ESI⁺): m/z = 297, 299 [M+H]⁺
(2) 3-Methyl-7-((E)-2-buten-1-yl)-8-brom-xanthin
   Massenspektrum (ESI⁺): m/z = 299, 301 [M+H]⁺
(3) 3-Cyclopropyl-7-(2-butin-1-yl)-8-brom-xanthin
   R_{f}-Wert: 0.45 (Reversed Phase DC-Fertigplatte (E. Merck), Acetonitril/Wasser/Trifluoressigsäure = 50:50:1)
   Massenspektrum (ESI⁺): m/z = 323, 325 [M+H]⁺

### Beispiel V

### 1-Chlormethyl-3-methyl-3.4-dihydro-isochinolin-3-yl-carbonsäuremethylester

hergestellt aus 2-(2-Chlor-acetylamino)-2-methyl-3-phenyl-propionsäuremethylester analog Das et al., Indian J. Chem. 1985, 24B, 1302.

R_{f}-Wert: 0.52 (Kieselgel, Petrolether/Essigester = 2:1)
Massenspektrum (ESI⁺): m/z = 252, 254 [M+H]⁺

### Beispiel VI

### 1-(2-{2-[2-(tert.-Butyloxycarbonylamino)-ethoxy]-phenyl}-2-oxo-ethyl)-3-methyl-7-(2-butin-1-yl)-8-[3-(tert.-butyloxycarbonyl-amino)-piperidin-1-yl]-xanthin

Zu 400 mg 1-[2-(2-Hydroxy-phenyl)-2-oxo-ethyl]-3-methyl-7-(2-butin-1-yl)-8-[3-(tert.-butyloxycarbonyl-amino)-piperidin-1-yl]-xanthin und 150 mg Kaliumcarbonat in 6 ml N,N-Dimethylformamid werden 187 mg 2-Brom-ethyl-carbaminsäure-tert.-butylester gegeben und das Reaktionsgemisch wird über Nacht bei 55°C gerührt. Dann werden nochmals 90 mg 2-Brom-ethyl-carbaminsäure-tert.-butylester zugegeben. Nach weiteren acht Stunden bei 55°C ist die Umsetzung vollständig. Das abgekühlte Reaktionsgemisch wird mit Wasser versetzt, der enstandene Niederschlag abgesaugt, mit Wasser nachgewaschen und getrocknet.
Ausbeute: 368 mg (73 % der Theorie)
Massenspektrum (ESI⁺): m/z = 694 [M+H]⁺

### Beispiel VII

### 1-[2-(2-Hydroxy-phenyl)-2-oxo-ethyl]-3-methyl-7-(2-butin-1-yl)-8-brom-xanthin

hergestellt durch Behandeln von 1-[2-(2-Methoxy-phenyl)-2-oxo-ethyl]-3-methyl-7-(2-butin-1-yl)-8-brom-xanthin mit Bortribromid in Gegenwart von Molsieb 4Å in Methylenchlorid bei 4°C.

Massenspektrum (ESI⁺): m/z = 431, 433 [M+H]⁺

### Beispiel VIII

### 1-[(1-Hydroxy-3-oxo-2,3-dihydro-1H-isoindol-1-yl)methyl]-3-methyl-7-(3-methyl-2-buten-1-yl)-8-[3-(tert.-butyloxycarbonyl-amino)-piperidin-1yl]-xanthin

Ein Gemisch aus 250 mg 1-[2-(2-Carboxy-phenyl)-2-oxo-ethyl]-3-methyl-7-(3-methyl-2-buten-1-yl)-8-[3-(tert.-butyloxycarbonyl-amino)-piperidin-1-yl]-xanthin, 404 mg Ammoniumcarbonat, 135 mg O-(Benzotriazol-1-yl)-N,N,N`,N`-tetramethyluroniumtetrafluoroborat, 57 mg Hydroxybenzotriazol und 59 µl Triethylamin in 3 ml Tetrahydrofuran wird acht Stunden bei Raumtemperatur gerührt. Zur Aufarbeitung wird das Reaktionsgemisch mit 30 ml Essigester verdünnt, mit 10 %iger Zitronensäurelösung, 10 %iger Kaliumcarbonatlösung und gesättigter Natriumchloridlösung gewaschen. Die organische Phase wird eingeengt und über eine Kieselgelsäule mit Methylenchlorid/Methanol (98:2 auf 80:20) chromatographiert. Als Hauptprodukt wird die cyclisierte Verbindung erhalten.
Ausbeute: 160 mg (64 % der Theorie)
R_{f}-Wert: 0.40 (Kieselgel, Methylenchlorid/Methanol = 9:1)
Massenspektrum (ESI⁺): m/z = 594 [M+H]⁺

### Beispiel IX

### 1-[2-(2-Carboxy-phenyl)-2-oxo-ethyl]-3-methyl-7-(3-methyl-2-buten-1-yl)-8-[3-(tert.-butyloxycarbonylamino)-piperidin-1yl]-xanthin

Ein Gemisch aus 2.60 g 1-[2-(2-Ethoxycarbonyl-phenyl)-2-oxo-ethyl]-3-methyl-7-(3-methyl-2-buten-1-yl)-8-[3-(tert.-butyloxycarbonylamino)-piperidin-1-yl]-xanthin und 8 ml 3 N Natronlauge in 25 ml Methanol wird zwei Stunden bei Raumtemperatur gerührt. Zur Aufarbeitung wird das Reaktionsgemisch mit 24 ml 1 N Salzsäure neutralisiert, durch Zugabe von 20 ml 10 %iger Zitronensäurelösung leicht angesäuert und mit Essigester extrahiert. Die vereinigten Extrakte werden mit gesättigter Natriumchloridlösung gewaschen, über Magnesiumsulfat getrocknet und eingeengt.
Ausbeute: 2.00 g (80 % der Theorie)
R_{f}-Wert: 0.49 (Kieselgel, Methylenchlorid/Methanol = 9:1)
Massenspektrum (ESI⁻): m/z = 593 [M-H]⁻

Analog Beispiel IX wird folgende Verbindung erhalten:
(1) 1-Carboxymethyl-3-methyl-7-(2-butin-1-yl)-8-[3-(tert.-butyloxycarbonyl-amino)-piperidin-1-yl]-xanthin
   (Die Esterspaltung erfolgt mit 4 M Kalilauge in einem Gemsich aus Methanol und Tetrahydrofuran.)
   Massenspektrum (ESI⁻): m/z = 473 [M-H]⁻

### Beispiel X

### 1-[(2-Oxo-2,3-dihydro-1H-benzo[e][1,4]diazepin-5-yl)methyl]-3-methyl-7-((E)-2-buten-1-yl)-8-[(R)-3-(tert.-butyloxycarbonylamino)piperidin-1-yl]-xanthin

Ein Gemisch aus 200 mg 1-{2-[2-(2-Chlor-acetylamino)-phenyl]-2-oxo-ethyl}-3-methyl-7-((E)-2-buten-1-yl)-8-[(*R*)-3-(tert.-butyloxycarbonylamino)-piperidin-1-yl]-xanthin, 5 ml konz. Ammoniak, 2 ml Tetrahydrofuran und 2 ml Methanol wird etwa eine Woche bei Raumtemperatur gerührt. Dann wird das dunkle Reaktionsgemisch auf eine Packung aus 14 g Extrelut gegeben und nach 20 Minuten gründlich mit Methylenchlorid ausgewaschen. Das Filtrat wird eingeengt und über eine Kieselgelsäule mit Essigester/Methanol (10:0 auf 8:2) als Laufmittel chromatographiert.
Ausbeute: 95 mg (51 % der Theorie)
R_{f}-Wert: 0.25 (Kieselgel, Cyclohäxan/Eäsigester = 2:8)

### Beispiel XI

### 1-{2-[2-(2-Chlor-acetylamino)-phenyl]-2-oxo-ethyl}-3-methyl-7-((E)-2-buten-1-yl)-8-[(R)-3-(tert.-butyloxycarbonylamino)-piperidin-1-yl]-xanthin

Zu 319 mg 1-[2-(2-Amino-phenyl)-2-oxo-ethyl]-3-methyl-7-((E)-2-buten-1-yi)-8-[(*R*)-3-(tert.-butyloxycarbonylamino)-piperidin-1-yl]-xanthin und 60 µl Pyridin in 1 ml Methylenchlorid werden 51 µl Bromacetylchlorid gegeben. Das Reaktionsgemisch wird zwei Stunden bei 35°C gerührt und nach Abkühlung auf Raumtemperatur mit 0.5 M Zitronensäure versetzt. Die organische Phase wird abgetrennt und die wässrige Phase mit Methylenchlorid extrahiert. Die vereinigten organischen Phasen werden eingeengt und über eine Kieselgelsäule mit Cyclohexan/Essigester (6:4) als Laufmittel chromatographiert.
Ausbeute: 210 mg (58 % der Theorie)
R_{f}-Wert: 0.50 (Kieselgel, Cyclohexan/Essigester/Isopropanol = 14:3:3)
Massenspektrum (ESI⁺): m/z = 628, 630 [M+H]⁺

Analog Beispiel XI werden folgende Verbindungen erhalten:
(1) *N*-(1-Benzyl-2,2,2-trifluor-ethyl)-2-chlor-acetamid
   (Die Umsetzung erfolgt mit Chloracetylchlorid in Diethylether in Gegenwart von Triethylamin.)
   R_{f}-Wert: 0.45 (Aluminiumoxid, Petrolether/Essigester = 5:1)
   Massenspektrum (ESI⁺): m/z = 266 [M+H]⁺
(2) 2-Chlor-*N*-(4-methyl-biphenyl-2-yl)-acetamid
   (Die Umsetzung erfolgt mit Chloracetylchlorid in Gegenwart von Diisopropylethylamin.)
   R_{f}-Wert: 0.82 (Kieselgel, Cyclohexan/Essigester = 1:1)
   Massenspektrum (ESI⁺): m/z = 260, 262 [M+H]⁺
(3) 2-Chlor-*N*-(6-methyl-biphenyl-2-yl)-acetamid
   (Die Umsetzung erfolgt mit Chloracetylchlorid in Gegenwart von Diisopropylethylamin.)
   R_{f}-Wert: 0.60 (Kieselgel, Cyclohexan/Essigester = 3:1)
   Massenspektrum (ESI⁺): m/z = 260, 262 [M+H]⁺
(4) 2-Chlor-*N*-(3-methyl-biphenyl-2-yl)-acetamid
   (Die Umsetzung erfolgt mit Chloracetylchlorid in Gegenwart von Diisopropylethylamin.)
   R_{f}-Wert: 0.45 (Kieselgel, Cyclohexan/Essigester = 3:1)

### Beispiel XII

### 1-[2-(2-Amino-phenyl)-2-oxo-ethyl]-3-methyl-7-((E)-2-buten-1-yl)-8-[(R)-3-(tert.-butyloxycarbonylamino)-piperidin-1-yl]-xanthin

hergestellt durch Reduktion von 6.34 g 1-[2-(2-Nitro-phenyl)-2-oxo-ethyl]-3-methyl-7-((E)-2-buten-1-yl)-8-[(*R*)-3-(tert.-butyloxycarbonylamino)-piperidin-1-yl]-xanthin mit 5.15 g Eisenpulver in einem Gemisch aus 260 ml Ethanol, 85 ml Wasser und 33 ml Eisessig unter Rückfluß.
Ausbeute: 5.38 g (90 % der Theorie)
Massenspektrum (ESI⁺): m/z = 552 [M+H]⁺

### Beispiel XIII

### 6-Chlormethyl-1,2,3,4-tetrahydro-phenanthridin-hydrochlorid

hergestellt durch Behandeln von 110 mg 6-Hydroxymethyl-1,2,3,4-tetrahydrophenanthridin mit 60 µl Thionylchlorid in 2.5 ml Methylenchlorid bei 0°C bis Raumtemperatur.
Aubeute: 140 mg (100 % der Theorie)
R_{f}-Wert: 0.50 (Kieselgel, Petrolether/Essigester = 5:1)
Massenspektrum (ESI⁺): m/z = 232, 234 [M+H]⁺

### Beispiel XIV

### 6-Hydroxymethyl-1,2,3,4-tetrahydro-phenanthridin

Zu einer Suspension aus 37 mg Lithiumborhydrid in 15 ml Tetrahydrofuran wird unter Eisbad-Kühlung innerhalb von fünf Minuten eine Lösung aus 350 mg 1,2,3,4-Tetrahydro-phenanthridin-6-yl-carbonsäure-ethylester in 10 ml Tetrahydrofuran getropft. Anschließend wird das Eisbad entfernt und das Reaktionsgemisch wird noch 2.5 Stunden bei Raumtemperatur nachgerührt. Zur Aufarbeitung werden unter Eisbad-Kühlung 2 ml 1 M Zitronensäure zur braunen Reaktionslösung gegeben. Das Gemisch wird mit 100 ml Essigester und 50 ml Wasser verrührt und mit 4 N Natronlauge auf pH 10 eingestellt. Die organische Phase wird abgetrennt, mit Wasser gewaschen, über Magnesiumsulfat getrocknet und eingeengt. Der Kolbenrückstand wird über eine Kieselgelsäule mit Essigester/Petrolether (1:4 auf 1:1) als Laufmittel chromatographiert.
Ausbeute: 120 mg (41 % der Theorie)
R_{f}-Wert: 0.40 (Kieselgel, Petrolether/Essigester = 2:1)
Massenspektrum (ESI⁺): m/z = 214 [M+H]⁺

### Beispiel XV

### 1,2,3,4-Tetrahydro-phenanthridin-6-yl-carbonsäure-ethylester

Analog dem von Gonsalves et al. beschriebenen Verfahren (Tetrahedron 1992, 48, 6821) wird eine Lösung aus 3.90 g 5,6,7,8-Tetrahydro-benzo[1,2,4]triazin-3-carbonsäure-ethylester (Sagi et al.; Heterocycles 1989, 29, 2253) in 20 ml Dioxan zum Rückfluß erhitzt. Dann werden mit Hilfe von zwei Tropftrichtern simultan 8.22 g Anthranilsäure und 7.02 g Isoamylnitrit, jeweils in 20 ml Dioxan gelöst, innerhalb von 25 Minuten zugetropft. Das Reaktionsgemisch wird noch weitere 30 Minuten unter Rückfluß erhitzt. Zur Aufarbeitung wird die abgekühlte tiefbraune Reaktionslösung mit 150 ml Diethylether verdünnt, mit 100 ml. 2 N Natronlauge und mit Wasser gewaschen, über Magnesiumsulfat getrocknet und eingeengt. Der braune, ölige Kolbenrückstand wird über eine Kieselgelsäule mit Essigester/Petrolether (20:80 auf 50:50) als Laufmittel chromatographiert. Das erhaltene Produkt ist noch etwas verunreinigt, wird aber ohne weitere Reinigung weiter umgesetzt.
Ausbeute: 380 mg (8 % der Theorie)
R_{f}-Wert: 0.55 (Kieselgel, Petrolether/Essigester = 2:1)
Massenspektrum (ESI⁺): m/z = 256 [M+H]⁺

### Beispiel XVI

### 1-Chlormethyl-3-trifluormethyl-3,4-dihydro-isochinolin

Zu 4.00 g warmer Polyphosphorsäure werden 0.74 ml Phosphoroxychlorid und 530 mg *N*-(1-Benzyl-2,2,2-trifluor-ethyl)-2-chlor-acetamid gegeben und das zähe Reaktionsgemisch wird 1.5 h bei 130°C gerührt. Nach Abkühlung auf Raumtemperatur wird das Reaktionsgemisch mit Eiswasser verrührt und abgesaugt. Der Filterkuchen wird in Essigester gelöst, die Lösung über Magnesiumsulfat getrocknet und eingeengt. Es bleibt ein weißer Feststoff zurück.
Ausbeute: 415 mg (84 % der Theorie)
R_{f}-Wert: 0.55 (Aluminiumoxid, Petrolether/Essigester = 10:1)
Massenspektrum (ESI⁺): m/z = 248, 250 [M+H]⁺

### Beispiel XVII

### 1-[(3,4-Dihydro-chinazolin-2-yl)methyl]-3-methyl-7-(2-butin-1-yl)-8-[3-(tert.-butyloxycarbonyl-amino)-piperidin-1-yl]-xanthin

Ein Gemisch aus 280 mg 1-[2-(2-Amino-benzylamino)-2-oxo-ethyl]-3-methyl-7-(2-butin-1-yl)-8-[3-(tert.-butyloxycarbonyl-amino)-piperidin-1-yl]-xanthin und 4 ml Eisessig wird zwei Stunden zum Sieden erhitzt. Anschließend wird das Reaktionsgemisch eingeengt und der Kolbenrückstand über eine Säule aus Aluminiumoxid (Aktivitätsstufe III) mit Methylenchlorid/Essigester/Methanol (5:5:0 auf 5:4:1) als Laufmittel gereingt. Dabei wird neben dem gewünschten 1-[(3,4-Dihydrochinazolin-2-yl)methyl]-3-methyl-7-(2-butin-1-yl)-8-[3-(tert.-butyloxycarbonyl-amino)-piperidin-1-yl]-xanthin auch bereits entschütztes 1-[(3,4-Dihydro-chinazolin-2-yl)methyl]-3-methyl-7-(2-butin-1-yl)-8-(3-amino-piperidin-1-yl)-xanthin erhalten.
Aubeute: 120 mg (44 % der Theorie)
Massenspektrum (ESI⁺): m/z = 561 [M+H]⁺

### Beispiel XVIII

### 1-[2-(2-Amino-benzylamino)-2-oxo-ethyl]-3-methyl-7-(2-butin-1-yl)-8-[3-(tert.-butyloxycarbonyl-amino)-pineridin-1-yl]-xanthin

Ein Gemisch aus 397 mg 1-Carboxymethyl-3-methyl-7-(2-butin-1-yl)-8-[3-(tert.-butyloxycarbonyl-amino)-piperidin-1-yl]-xanthin, 110 mg 2-Amino-benzylamin und 460 µl Diisopropylethylamin in 3 ml N,N-Dimethylformamid wird mit 272 mg (Benzotriazol-1-yl)-N-tetramethyl-uronium-tetrafluoroborat versetzt und zwei Stunden bei Raumtemperatur gerührt. Anschließend wir das Reaktionsgemisch eingeengt, der Rückstand mit 15 ml 1 M Natronlauge verrieben und abgesaugt. Der Filterkuchen wird mit wenig Ethanol und Diethylether gewaschen und getrocknet.
Ausbeute: 400 mg (83 % der Theorie)
R_{f}-Wert: 0.68 (Kieselgel, Methylenchlorid/Methanol = 9:1)
Massenspektrum (ESI⁺): m/z = 579 [M+H]⁺

### Beispiel XIX

### 1-[(3-Methyl-3,4-dihydro-chinazolin-2-yl)methyl]-3-methyl-7-(2-butin-1-yl)-8-[3-(tert.-butyloxycarbonyl-amino)-piperidin-1-yl]-xanthin

Zu 400 mg 1-Cyanomethyl-3-methyl-7-(2-butin-1-yl)-8-[3-(tert.-butyloxycarbonylamino)-piperidin-1-yl]-xanthin in 5 ml Methanol werden 0.5 ml 1 M Natriummethanolat-Lösung gegeben. Das Reaktionsgemisch wird zwei Stunden bei Raumtemperatur gerührt, dann werden weitere 150 µl 1 M Natriummethanolat-Lösung zugegeben. Nach weiteren zwei Stunden ist die Umsetzung zum Iminoester vollständig und das Reaktionsgemisch wird mit 1 M Essigsäure in Methanol neutralisiert. Dann wird eine Lösung aus 130 mg 2-Methylaminomethyl-phenylamin in 3 ml Methanol zugegeben und das Reaktionsgemisch drei Stunden unter Rückfluß erhitzt. Anschließend wird das Methanol abdestilliert und der Rückstand mit Wasser verrührt, abgesaugt und getrocknet.
Ausbeute: 250 mg (50 % der Theorie)
Massenspektrum (ESI⁺): m/z = 575 [M+H]⁺

Analog Beispiel XIX wird folgende Verbindung erhalten:
(1) 1-[(1*H*-Perimidin-2-yl)methyl]-3-methyl-7-(2-butin-1-yl)-8-[(*R*)-3-(tert.-butyloxycarbonyl-amino)-piperidin-1-yl]-xanthin
Massenspektrum (ESI⁺): m/z = 597 [M+H]⁺

### Beispiel XX

### 3-Cyclopropyl-8-brom-xanthin

Zu einem Gemisch aus 3.67 g 3-Cyclopropyl-xanthin und 3.40 g Kaliumcarbonat in 60 ml Acetonitril werden bei 60 °C Ölbadtemperatur langsam 1.08 ml Brom getropft. Das Reaktionsgemisch wird sechs Stunden bei dieser Temperatur gerührt, dann werden weitere 100 µl Brom zugegeben. Nach weiteren drei Stunden wird das Acetonitril im Vakuum abdestilliert und der Rückstand in 100 ml Wasser gelöst. Dann werden 10 ml gesättigte Natriumthiosulfat-Lösung zugegeben und es wird mit Essigester extrahiert. Die wässrige Phase wird mit 1 M Salzsäure angesäuert, worauf sich ein feiner Niederschlag bildet. Der Niederschlag wird abgesaugt, mit Wasser und Diethylether gewaschen und im Umlufttrockenschrank bei 80°C getrocknet.
Ausbeute: 3.36 g (65 % der Theorie)
R_{f}-Wert: 0.65 (Reversed Phase DC-Fertigplatte (E. Merck), Acetonitril/Wasser/Trifluoressigsäure = 50:50:1)
Massenspektrum (ESI⁺): m/z= 271, 273 [M+H]⁺

### Beispiel XXI

### 6-Chlormethyl-8-methyl-phenanthridin

600 g 2-Chlor-*N*-(4'-methyl-biphenyl-2-yl)-acetamid werden in 3 ml Phosporoxychlorid etwa 6 Stunden auf 100 °C erhitzt. Anschließend wird das Phosporoxychlorid abdestilliert. Der Rückstand wird in Wasser und Essigester suspendiert und unter Eisbad-Kühlung mit 3 M Natronlauge neutralisiert. Die wässrige Phase wird mit Essigester extrahiert und die vereinigten organischen Phasen werden mit gesättigter Natriumchlorid-Lösung gewaschen, über Magnesiumsulfat getrocknet und eingeengt. Der Rückstand wird mit Diisopropylether verrieben, abgesaugt und getrocknet.
Ausbeute: 160 mg (29 % der Theorie)
R_{f}-Wert: 0.45 (Kieselgel, Cyclohexan/Essigester = 1:1)
Massenspektrum (ESI⁺): m/z = 242, 244 [M+H]⁺

Analog Beispiel XXI werden folgende Verbindungen erhalten:
(1) 6-Chlormethyl-1-methyl-phenanthridin
   Massenspektrum (ESI⁺): m/z = 242, 244 [M+H]⁺
(2) 6-Chlormethyl-4-methyl-phenanthridin
   Massenspektrum (ESI⁺): m/z = 242, 244 [M+H]⁺

### Beispiel XXII

### 1-[(Indolizin-2-yl)methyl]-3-methyl-7-(2-butin-1-yl)-8-brom-xanthin

Zu einem Gemisch aus 594 mg 3-Methyl-7-(2-butin-1-yl)-8-brom-xanthin, 353 mg (Indolizin-2-yl)-methanol und 826 mg Triphenylphosphin in 30 ml Tetrahydrofuran werden 0.61 ml Diisopropylazodicarboxylat gegeben. Das Reaktionsgemisch wird zwei Stunden bei Raumtemperatur gerührt. Zur Aufarbeitung wird es mit Methylenchlorid verdünnt, auf 6 g Kieselgel aufgezogen und über eine Kieselgelsäule mit Petrolether/Essigester (7:3 auf 1:5) als Laufmittel chromatographiert.
Ausbeute: 405 mg (48 % der Theorie)
R_{f}-Wert: 0.62 (Kieselgel, Petrolether/Essigester = 1:1)
Massenspektrum (ESI⁺): m/z = 426, 428 [M+H]⁺

### Beispiel XXIII

### 6-Methyl-benzo[c][1,8]naphthyridin

Zu einem Gemisch aus 960 mg Eisen(II)sulfat-heptahydrat, 12.00 g 3-Nitrobenzosulfonsäure-Natriumsalz, 15 ml konz. Schwefelsäure und 1.70 g Borsäure werden unter im Eisbad-Kühlung 8.7 ml Glycerin und 4.38 g 3-Amino-1-methylisochinolin gegeben. Die zähe, klebrige Masse wird auf ca. 55°C erwärmt, mit 15 ml Wasser versetzt und anschließend drei Stunden bei 140°C gerührt. Das abgekühtte Reaktionsgemisch wird mit etwas Eis verdünnt, unter Eisbad-Kühlung mit 15 N Natronlauge alkalisch gestellt und mit Methylenchlorid extrahiert. Die vereinigten organischen Phasen werden mit gesättigter Natriumchlorid-LÖsung gewaschen, über Magnesiumsulfat getrocknet und eingeengt. Der Kolbenrückstand wird über eine Kieselgesäule mit Essigester/Methanol (99:1 auf 94:6) als Laufmittel chromatographiert. Das so erhaltene Rohprodukt wird mit tert.-Butylmethylether und etwas Essigester verrührt, abgesaugt und getrocknet.
Ausbeute: 2.05 g (38 % der Theorie)
R_{f}-Wert: 0.15 (Kieselgel, Essigester)
Massenspektrum (ESI⁺): m/z = 195 [M+H]⁺

Analog Beispiel XXIII wird folgende Verbindung erhalten:
(1) 6-Methyl-benzo[*c*][1,5]naphthyridin
R_{f}-Wert: 0.52 (Kieselgel, Methylenchlorid/Methanol = 95:5)
Massenspektrum (ESI⁺): m/z = 195 [M+H]⁺

### Beispiel XXIV

### 6-Methyl-benzo[f]chinoxalin

170 mg 4-Methyl-naphthalin-1,2-diamin und 114 µl Glyoxal werden in einem Gemisch aus 2 ml Wasser und 2 ml Ethanol eine halbe Stunde bei 75°C gerührt. Zur Aufarbeitung wird das abgekühlte Reaktionsgemisch mit Methylenchlorid und Wasser verdünnt. Die organische Phase wird mit gesättigter Natriumchlorid-Lösung gewaschen, über Magnesiumsulfat getrocknet und eingeengt. Das Rohprodukt wird chromatographisch über eine Kieselgelsäule mit Methylenchlorid/Methanol (100:0 auf 99:1) als Laufmittel gereinigt.
Ausbeute: 140 mg (73 % der Theorie)
R_{f}-Wert: 0.84 (Kieselgel, Essigester)
Massenspektrum (ESI⁺): m/z = 195 [M+H]⁺

### Beispiel XXV

### 2-Chlormethyl-imidazo[2,1-a]isochinolin

1.47 g 1-Amino-isochinolin und 635 mg 1,3-Dichloraceton werden in 10 ml Acetonitril eine Stunde unter Rückfluß erhitzt. Zur Aufarbeitung wird das Reaktionsgemisch mit Methanol versetzt, auf ca. 5 g Kieselgel aufgezogen und über eine Kieselgelsäule mit Methylenchlorid/Methanol (98:2 auf 96:4) als Laufmittel chromatographiert.
Ausbeute: 420 mg (39 % der Theorie)
R_{f}-Wert: 0.65 (Kieselgel, Methylenchlorid/Methanol = 95:5)
Massenspektrum (ESI⁺): m/z = 217, 219 [M+H]⁺

Analog Beispiel XXV wird folgende Verbindung erhalten:
(1) 2-Chlormethyl-imidazo[1,2-a]isochinolin
R_{f}-Wert: 0.64 (Kieselgel, Methylenchlorid/Methanol = 95:5)
Massenspektrum (ESI⁺): m/z = 217, 219 [M+H]⁺

### Herstellung der Endverbindungen:

### Beispiel 1

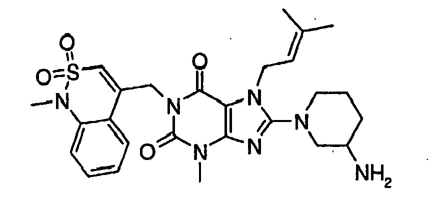

1-[(1-Methyl-2,2-dioxo-1*H*-benzo[*c*][1,2]thiazin-4-yl)methyl]-3-methyl-7-(3-methyl-2-buten-1-yl)-8-(3-amino-peridin-1-yl)-xanthin
Zu 340 mg 1-[(1-Methyl-2,2-dioxo-1*H*-benzo[*c*][1,2]thiazin-4-yl)methyl]-3-methyl-7-(3-methyl-2-buten-1-yl)-8-[3-(tert.-butyloxycarbonylamino)-piperidin-1-yl]-xanthin in 15 ml Methylenchlorid werden 3.5 ml isopropanolische Salzsäure (5-6 M) gegeben und das Reaktionsgemisch wird drei Stunden bei Raumtemperatur gerührt. Zur Aufarbeitung wird es mit Wasser und Methylenchlorid verdünnt und mit 18 ml 1N Natronlauge versetzt. Die wässrige Phase wird mit Methylenchlorid extrahiert und die vereinigten organischen Phasen werden mit Wasser gewaschen, über Magnesiumsulfat getrocknet und eingeengt. Der gelbliche, schaumige Kolbenrückstand wird mit tert.-Butylmethylether und wenig Diethylether verrührt, der entstandene helle Niederschlag abgesaugt und in der Trockenpistole bei 60°C getrocknet.
Ausbeute: 220 mg (77 % der Theorie)
Schmelzpunkt: 205-208°C (Zersetzung)
Massenspektrum (ESI⁺): m/z = 540 [M+H]⁺

Analog Beispiel 1 werden folgende Verbindungen erhalten:
(1) 1-[(3-Methoxycarbonyl-3-methyl-3,4-dihydro-isochinolin-1-yl]methyl]-3-methyl-7-(2-butin-1-yl)-8-(3-amino-piperidin-1-yl)-xanthin 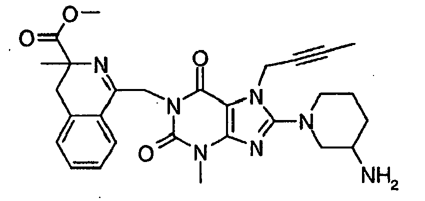 R_{f}-Wert: 0.42 (Kieselgel, Methylenchlorid/Methanol/konz. wässriges Ammoniak = 90:10:1)
   Massenspektrum (ESI⁺): m/z = 532 [M+H]⁺
(2) 1-[(2-Oxo-2,3-dihydro-1H-benzo[*e*][1,4]diazepin-5-yl)methyl]-3-methyl-7-((E)-2-buten-1-yl)-8-((*R*)-3-amino-piperidin-1-yl)-xanthin

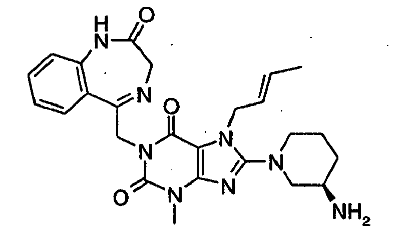

### (Durchführung mit Trifluoressigsäure in Methylenchlorid)

R_{f}-Wert: 0.50 (Reversed Phase DC-Fertigplatte (E. Merck), Acetonitril/Wasser/Trifluoressigsäure = 50:50:0.1)
Massenspektrum (ESI⁺): m/z = 491 [M+H]⁺
(3) 1-[(Phenanthridin-6-yl)methyl]-3-methyl-7-(2-butin-1-yl)-8-(3-amino-piperidin-1-yl)-xanthin-dihydrochlorid 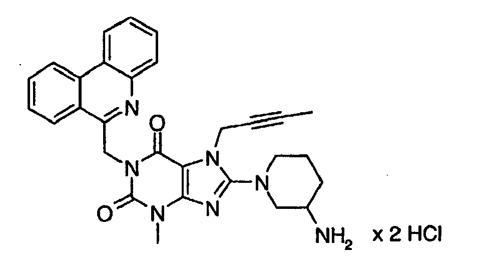 R_{f}-Wert: 0.55 (Reversed Phase DC-Fertigplatte (E. Merck), Acetonitril/Wasser/Trifluoressigsäure = 50:50:1)
   Massenspektrum (ESI⁺): m/z = 508 [M+H]⁺
(4) 1-[(1,2,3,4-Tetrahydro-phenanthridin-6-yl)methyl]-3-methyl-7-(2-butin-1-yl)-8-(3-amino-piperidin-1-yl)-xanthin x Trifluoressigsäure

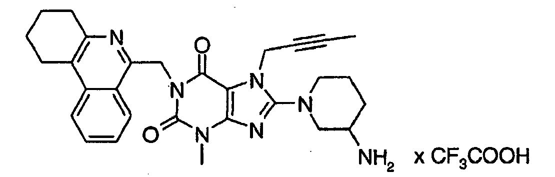

### (Durchführung mit Trifluoressigsäure in Methylenchlorid)

R_{f}-Wert: 0.75 (Aluminiumoxid, Methylenchlorid/Methanol = 10:1) Massenspektrum (ESI⁺): m/z = 512 [M+H]⁺
(5) 1-[(11*H*-Dibenzo[*b*,*e*]azepin-6-yl)methyl]-3-methyl-7-(2-butin-1-yl)-8-(3-aminopiperidin-1-yl)-xanthin 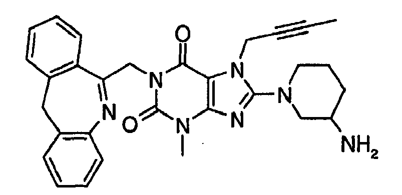
R_{f}-Wert: 0.45 (Reversed Phase DC-Fertigplatte (E. Merck), Acetonitril/Wasser/Trifluoressigsäure = 50:50:1)

Massenspektrum (ESI⁺): m/z = 522 [M+H]⁺ (6) 1-[(Dibenzo[*b*,*f*][1,4]oxazepin-11-yl)methyl]-3-methyl-7-(2-butin-1-yl)-8-(3-aminopiperidin-1-yl)-xanthin 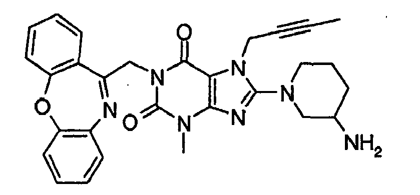 Massenspektrum (ESI⁺): m/z = 524 [M+H]⁺ (7) 1-[(3-Trifluormethyl-3,4-dihydro-isochinolin-1-yl)methyl]-3-methyl-7-(2-butin-1-yl)-8-(3-amino-piperidin-1-yl)-xanthin x Trifluoressigsäure

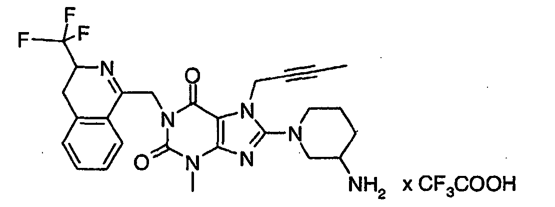

### (Durchführung mit Trifluoressigsäure in Methylenchlorid)

R_{f}-Wert: 0.30 (Kieselgel, Methylenchlorid/Methanol = 10:1)
Massenspektrum (ESI⁺): m/z = 528 [M+H]⁺
(8) 1-[(Dibenzo[*b*,*f*][1,4]oxazepin-11-yl)methyl]-3-methyl-7-(2-butin-1-yl)-8-((*R*)-3-amino-piperidin-1-yl)-xanthin 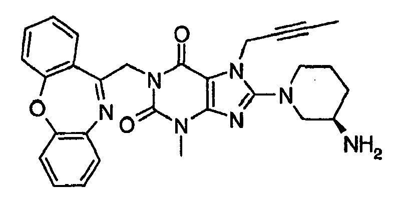 Massenspektrum (ESI⁺): m/z = 524 [M+H]⁺
   Schmelzpunkt: 128°C
(9) 1-[(3,3-D)methyl-3,4-dihydro-isochinolin-1-yl)methyl]-3-methyl-7-(2-butin-1-yl)-8-(3-amino-piperidin-1-yl)-xanthin 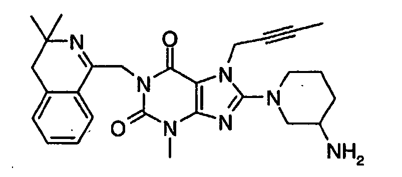
R_{f}-Wert: 0.55 (Reversed Phase DC-Fertigplatte (E. Merck), Acetonitril/Wasser/Trifluoressigsäure = 50:50:1) Massenspektrum (ESI⁺): m/z = 488 [M+H]⁺
(10) 1-[(3,4-Dihydro-chinazolin-2-yl)methyl]-3-methyl-7-(2-butin-1-yl)-8-(3-aminopiperidin-1-yl)-xanthin

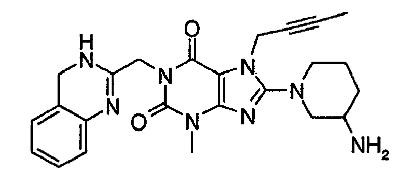

### (Durchführung mit Trifluoressigsäure in Methylenchlorid)

Massenspektrum (ESI⁺): m/z = 461 [M+H]⁺
(11) 1-[(3-Methyl-3,4-dihydro-chinazolin-2-yl)methyl]-3-methyl-7-(2-butin-1-yl)-8-(3-amino-piperidin-1-yl)-xanthin x Trifluoressigsäure

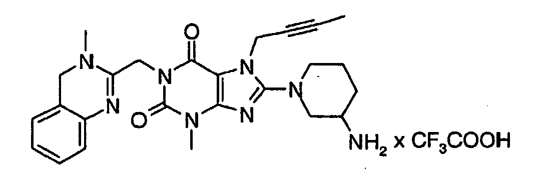

### (Durchführung mit Trifluoressigsäure in Methylenchlorid)

Massenspektrum (ESI⁺): m/z = 475 [M+H]⁺ (12) 1-[(5-Methyl-5*H*-dibenzo[*b*,*e*][1,4]diazepin-11-yl)methyl]-3-methyl-7-(2-butin-1-yl)-8-(3-amino-piperidin-1-yl)-xanthin 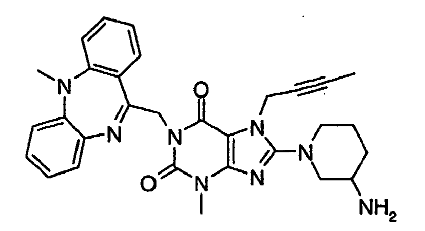 R_{f}-Wert: 0.45 (Reversed Phase DC-Fertigplatte (E. Merck), Acetonitril/Wasser/Trifluoressigsäure = 50:50:1)
Massenspektrum (ESI⁺): m/z = 537 [M+H]⁺ (13) 1-[(8-Methyl-dibenzo[*b*,*f*][1,4]oxazepin-11-yl)methyl]-3-methyl-7-(2-butin-1-yl)-8-(3-amino-piperidin-1-yl)-xanthin 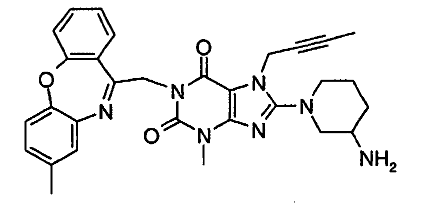 R_{f}-Wert: 0.60 (Reversed Phase DC-Fertigplatte (E. Merck), Acetonitril/Wasser/Trifluoressigsäure = 50:50:1)
Massenspektrum (ESI⁺): m/z = 538 [M+H]⁺ (14) 1-[(2-Methyl-dibenzo[*b*,*f*][1,4]oxazepin-11-yl)methyl]-3-methyl-7-(2-butin-1-yl)-8-(3-amino-piperidin-1-yl)-xanthin 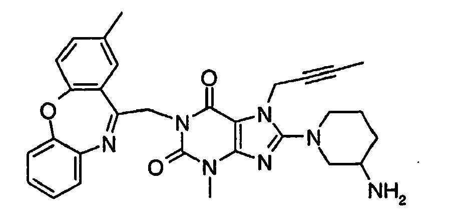 R_{f}-Wert: 0.55 (Reversed Phase DC-Fertigplatte (E. Merck), Acetonitril/Wasser/Trifluoressigsäure = 50:50:1)
Massenspektrum (ESI⁺): m/z = 538 [M+H]⁺ (15) 1-[(Benzo[1,2,5]oxadiazol-5-yl)methyl]-3-methyl-7-(2-butin-1-yl)-8-(3-aminopiperidin-1-yl)-xanthin 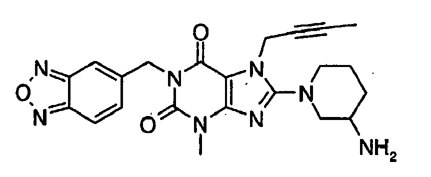 R_{f}-Wert: 0.38 (Kieselgel, Methylenchlorid/Methanol = 9:1)
Massenspektrum (ESI⁺): m/z = 449 [M+H]⁺ (16) 1-[(2-Chlor-dibenzo[*b,f*][1,4]oxazepin-11-yl)methyl]-3-methyl-7-(2-butin-1-yl)-8-(3-amino-piperidin-1-yl)-xanthin 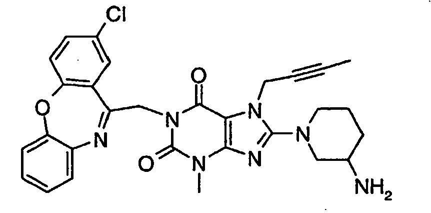 R_{f}-Wert: 0.50 (Reversed Phase DC-Fertigplatte (E. Merck), Acetonitril/Wasser/Trifluoressigsäure = 50:50:1)
Massenspektrum (ESI⁺): m/z = 558, 560 [M+H]⁺ (17) 1-[(Phenanthridin-6-yl)methyl]-3-cyclopropyl-7-(2-butin-1-yl)-8-(3-aminopiperidin-1-yl)-xanthin 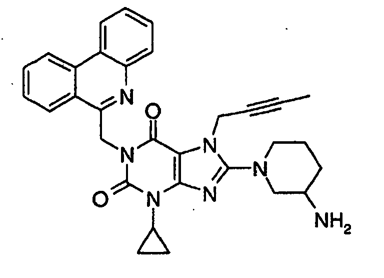 R_{f}-Wert: 0.50 (Reversed Phase DC-Fertigplatte (E. Merck), Acetonitril/Wasser/Trifluoressigsäure = 50:50:1)
Massenspektrum (ESI⁺): m/z = 534 [M+H]⁺ (18) 1-[(8-Methyl-phenanthridin-6-yl)methyl]-3-methyl-7-(2-butin-1-yl)-8-(3-aminopiperidin-1-yl)-xanthin 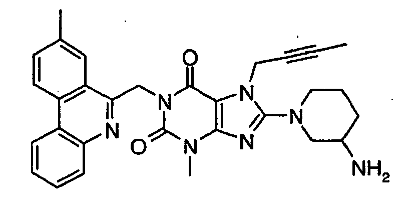 Schmelzpunkt: 200-205°C
Massenspektrum (ESI⁺): m/z = 522 [M+H]⁺ (19) 1-[(Phenanthridin-6-yl)methyl]-3-methyl-7-(2-butin-1-yl)-8-((*S*)-3-amino-piperidin-1-yl)-xanthin 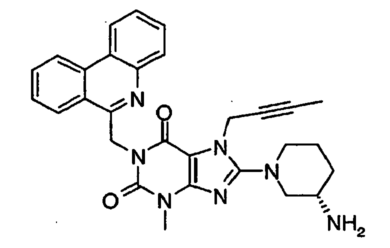 R_{f}-Wert: 0.55 (Reversed Phase DC-Fertigplatte (E. Merck), Acetonitril/Wasser/Trifluoressigsäure = 50:50:1)
Massenspektrum (ESI⁺): m/z = 508 [M+H]⁺ (20) 1-[(Phenanthridin-6-yl)methyl]-3-cyclopropyl-7-(2-butin-1-yl)-8-((*R*)-3-aminopiperidin-1-yl)-xanthin 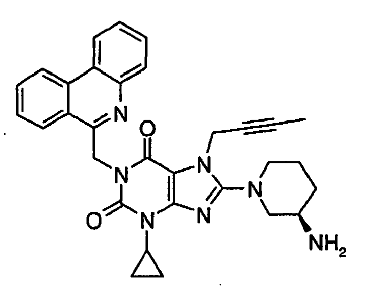 R_{f}-Wert: 0.50 (Reversed Phase DC-Fertigplatte (E. Merck), Acetonitril/Wasser/Trifluoressigsäure = 50:50:1)
Massenspektrum (ESI⁺): m/z = 534 [M+H]⁺ (21) 1-[(Dibenzofuran-2-yl)methyl]-3-methyl-7-(2-butin-1-yl)-8-((*R*)-3-amino-piperidin-1-yl)-xanthin 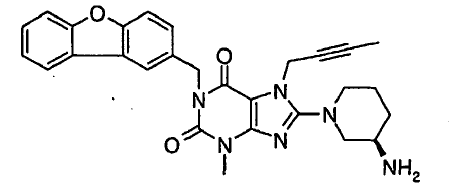 R_{f}-Wert: 0.40 (Reversed Phase DC-Fertigplatte (E. Merck), Acetonitril/Wasser/Trifluoressigsäure = 50:50:1)
Massenspektrum (ESI⁺): m/z = 497 [M+H]⁺ (22) 1-[(1-Methyl-phenanthridin-6-yl)methyl]-3-methyl-7-(2-butin-1-yl)-8-((*R*)-3-aminopiperidin-1-yl)-xanthin 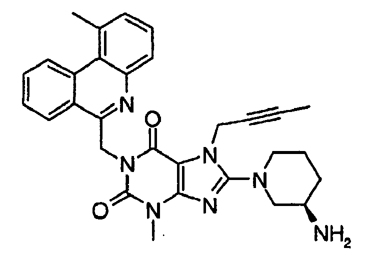 R_{f}-Wert: 0.50 (Reversed Phase DC-Fertigplatte (E. Merck), Acetonitril/Wasser/Trifluoressigsäure = 50:50:1)
Massenspektrum (ESI⁺): m/z = 522 [M+H]⁺ (23) 1-[(4-Methyl-phenanthridin-6-yl)methyl]-3-methyl-7-(2-butin-1-yl)-8-((*R*)-3-aminopiperidin-1-yl)-xanthin 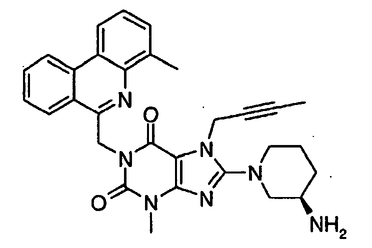 R_{f}-Wert: 0.40 (Reversed Phase DC-Fertigplatte (E. Merck), Acetonitril/Wasser/Trifluoressigsäure = 50:50:1)
Massenspektrum (ESI⁺): m/z = 522 [M+H]⁺ (24) 1-[(Indolizin-2-yl)methyl]-3-methyl-7-(2-butin-1-yl)-8-(3-amino-piperidin-1-yl)-xanthin 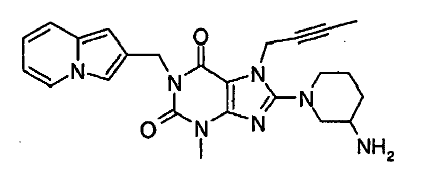 R_{f}-Wert: 0.47 (Kieselgel, Methylenchlorid/Methanol/konz. wässriges Ammoniak = 90:10:1)
Massenspektrum (ESI⁺): m/z = 446 [M+H]⁺ (25) 1-[(Benzo[*h*][1,6]naphthyridin-5-yl)methyl]-3-methyl-7-(2-butin-1-yl)-8-(3-aminopiperidin-1-yl)-xanthin 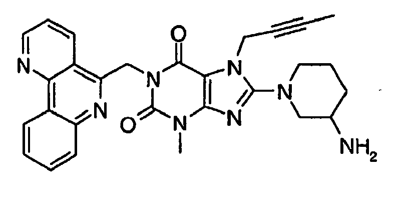 R_{f}-Wert: 0.49 (Kieselgel, Methylenchlorid/Methanol/konz. wässriges Ammoniak = 90:10:1)
Massenspektrum (ESI⁺): m/z = 509 [M+H]⁺ (26) 1-[(Pyrazolo[1,5-c]chinazolin-5-yl)methyl]-3-methyl-7-(2-butin-1-yl)-8-(3-aminopiperidin-1-yl)-xanthin 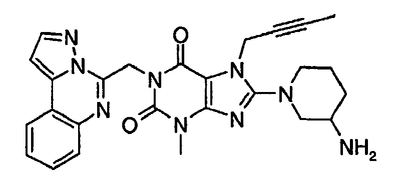 R_{f}-Wert: 0.46 (Kieselgel, Methylenchlorid/Methanol/konz. wässriges Ammoniak = 90:10:1)
Massenspektrum (ESI⁺): m/z = 498 [M+H]⁺ (27) 1-[(Benzo[*c*][1,8]naphthyridin-6-yl)methyl]-3-methyl-7-(2-butin-1-yl)-8-(3-aminopiperidin-1-yl)-xanthin 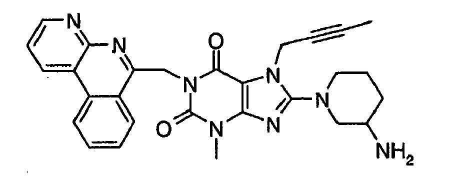 R_{f}-Wert: 0.48 (Kieselgel, Methylenchlorid/Methanol/konz. wässriges Ammoniak = 90:10:1)
Massenspektrum (ESI⁺): m/z = 509 [M+H]⁺ (28) 1-[(Benzo[*c*][1,5]naphthyridin-6-yl)methyl]-3-methyl-7-(2-butin-1-yl)-8-((*R*)-3-amino-piperidin-1-yl)-xanthin 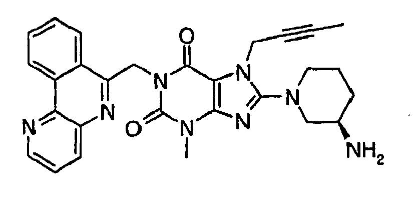 R_{f}-Wert: 0.51 (Kieselgel, Methylenchlorid/Methanol/konz. wässriges Ammoniak = 90:10:1)
Massenspektrum (ESI⁺): m/z = 509 [M+H]⁺ (29) 1-[(1*H*-Perimidin-2-yl)methyl]-3-methyl-7-(2-butin-1-yl)-8-((*R*)-3-amino-piperidin-1-yl)-xanthin 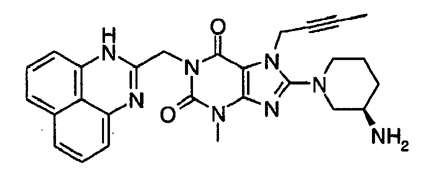 R_{f}-Wert: 0.47 (Kieselgel, Methylenchlorid/Methanol/konz. wässriges Ammoniak = 90:10:1)
Massenspektrum (ESI⁺): m/z = 497 [M+H]⁺ (30) 1-[(Benzo[*f*]chinoxalin-6-yl)methyl]-3-methyl-7-(2-butin-1-yl)-8-((*R*)-3-aminopiperidin-1-yl)-xanthin 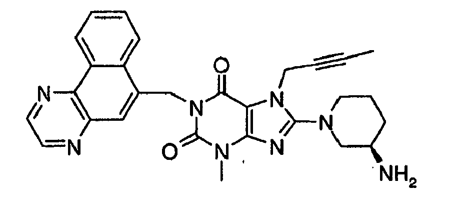 R_{f}-Wert: 0.50 (Kieselgel, Methylenchlorid/Methanol/konz. wässriges Ammoniak = 90:10:1)
Massenspektrum (ESI⁺): m/z = 509 [M+H]⁺ (31) 1-[(Imidazo[2,1-a]isochinolin-2-yl)methyl]-3-methyl-7-(2-butin-1-yl)-8-(3-aminopiperidin-1-yl)-xanthin 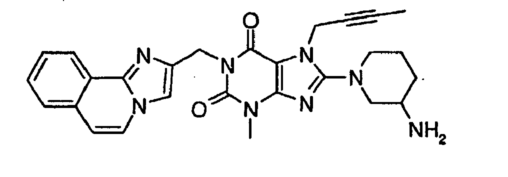 R_{f}-Wert: 0.54 (Kieselgel, Methylenchlorid/Methanol/konz. wässriges Ammoniak = 90:10:1)
Massenspektrum (ESI⁺): m/z = 497 [M+H]⁺ (32) 1-[(Imidazo[1,2-a]isochinolin-2-yl)methyl]-3-methyl-7-(2-butin-1-yl)-8-(3-aminopiperidin-1-yl)-xanthin 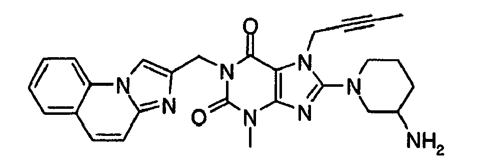 Schmelzpunkt: 194-198.5°C
Massenspektrum (ESI⁺): m/z = 497 [M+H]⁺ (33) 1-[(Phenanthridin-6-yl)methyl]-3-methyl-7-(2-butin-1-yl)-8-((*R*)-3-amino-piperidin-1-yl)-xanthin 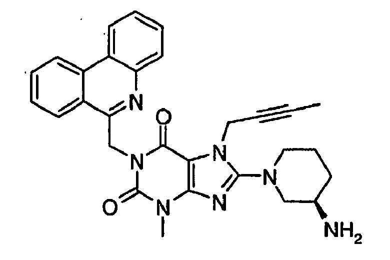 R_{f}-Wert: 0.55 (Reversed Phase DC-Fertigplatte (E. Merck), Acetonitril/Wasser/Trifluoressigsäure = 50:50:1)
Massenspektrum (ESI⁺): m/z = 508 [M+H]⁺

### Beispiel 2

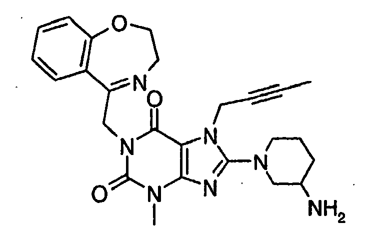

1-[(2,3-Dihydro-benzo[*f*][1,4]oxazepin-5-yl)methyl]-3-methyl-7-(2-butin-1-yl)-8-(3-amino-piperidin-1-yl)-xanthin
Zu 368 mg 1-(2-{2-[2-(tert.-Butyloxycarbonylamino)-ethoxy]-phenyl}-2-oxo-ethyl)-3-methyl-7-(2-butin-1-yl)-8-[3-(tert.-butyloxycarbonyl-amino)-piperidin-1-yl]-xanthin in 7 ml Methylenchlorid werden unter Eisbad-Kühlung 1.15 ml Trifluoressigsäure gegeben. Das Reaktionsgemisch wird etwa drei Stunden bei Raumtemperatur gerührt und anschließend auf gekühlte Kaliumcarbonatlösung gegeben. Die organische Phase wird mit gesättigter Natriumchloridlösung gewaschen, über Magnesiumsulfat getrocknet und eingeengt. Das Rohprodukt wird über eine Kieselgelsäule mit Methylenchlorid/Methanol (10:0 auf 7:3) als Laufmittel gereinigt.
Ausbeute: 75 mg (30 % der Theorie)
R_{f}-Wert: 0.20 (Kieselgel, Methylenchlorid/Methanol = 9:1)
Massenspektrum (ESI⁺): m/z = 476 [M+H]⁺

### Beispiel 3

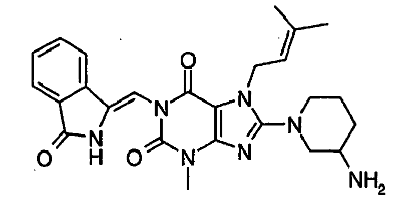

1-[(3-Oxo-2,3-dihydro-isoindol-1-yliden)methyl]-3-methyl-7-(3-methyl-2-buten-1-yl)-8-(3-amino-piperidin-1-yl)-xanthin
150 mg 1-[(1-Hydroxy-3-oxo-2,3-dihydro-1*H*-isoindol-1-yl)methyl]-3-methyl-7-(3-methyl-2-buten-1-yl)-8-[3-(tert.-butyloxycarbonyl-amino)-piperidin-1-yl]-xanthin werden vier Stunden in einem Gemisch aus 0.4 ml Trifluoressigsäure und 1.2 ml Methylenchlorid gerührt. Zur Aufarbeitung wird das Reaktionsgemisch mit 30 ml Methylenchlorid verdünnt, mit 10 ml 10 %iger Kaliumcarbonatlösung versetzt und und kräftig durchgerührt. Die organische Phase wird abgetrennt, über Magnesiumsulfat getrocknet und eingeengt.
Ausbeute: 50 mg (42 % der Theorie)
R_{f}-Wert: 0.56 (Reversed Phase DC-Fertigplatte (E. Merck), Acetonitril/Wasser/Trifluoressigsäure = 50:50:1)
Massenspektrum (ESI⁺): m/z = 476 [M+H]⁺

Analog den vorstehenden Beispielen und anderen literaturbekannten Verfahren können auch die folgenden Verbindungen erhalten werden:

| Nr. | Name | Strukturformel |
|---|---|---|
| (1) | 1-[(1-Methyl-1,4-dihydro-chinazolin-2-yl)methyl]-3-methyl-7-(2-butin-1-yl)-8-(3-amino-piperidin-1-yl)-xanthin | 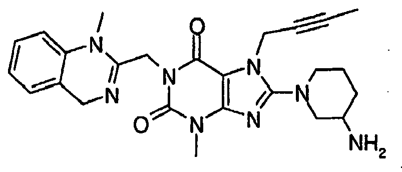 |
| (2) | 1-[(3,4-Dihydro-chinazolin-2-yl)methyl]-3-methyl-7-(2-butin-1-yl)-8-(3-amino-piperidin-1-yl)-xanthin | 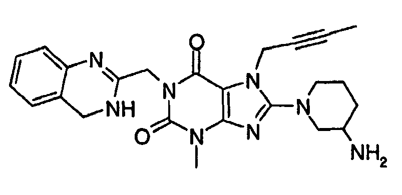 |
| (3) | 1-[(3-Methyl-3,4-dihydro-chinazolin-2-yl)methyl]-3-methyl-7-(2-butin-1-yl)-8-(3-amino-piperidin-1-yl)-xanthin | 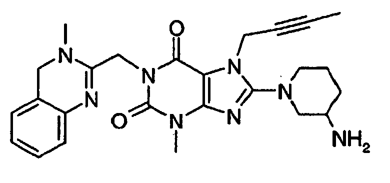 |
| (4) | 1-[(3,4-Dihydro-isochinolin-1-yl)methyl]-3-methyl-7-(2-butin-1-yl)-8-(3-amino-piperidin-1-yl)-xanthin | 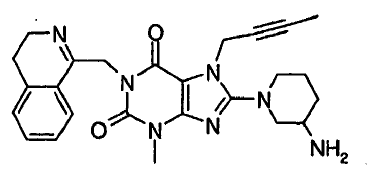 |
| (5) | 1-[(3,3-Dimethyl-3,4-dihydro-isochinolin-1-yl)methyl]-3-methyl-7-(2-butin-1-yl)-8-(3-amino-piperidin-1-yl)-xanthin | 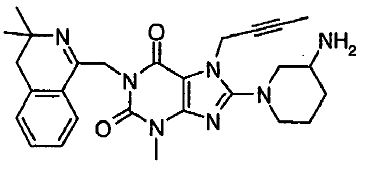 |
| (6) | 1-[(4,4-Dimethyl-3,4-dihydro-isochinolin-1-yl)methyl]-3-methyl-7-(2-butin-1-yl)-8-(3-amino-piperidin-1-yl)-xanthin | 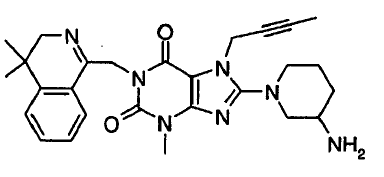 |
| (7) | 1-[(1*H*-Benzo[*d*][1,2]oxazin-4-yl)methyl]-3-methyl-7-(2-butin-1-yl)-8-(3-amino-piperidin-1-yl)-xanthin | 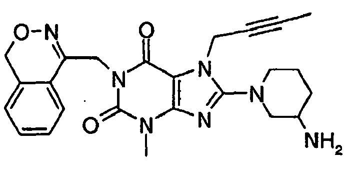 |
| (8) | 1-[(1-Oxo-1*H*-benzo[*d*][1,2]oxazin-4-yl)methyl]-3-methyl-7-(2-butin-1-yl)-8-(3-amino-piperidin-1-yl)-xanthin | 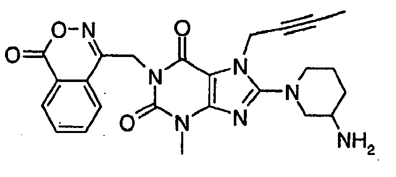 |
| (9) | 1-[(4*H*-Benzo[*e*][1,3]oxazin-2-yl)methyl]-3-methyl-7-(2-butin-1-yl)-8-(3-amino-piperidin-1-yl)-xanthin | 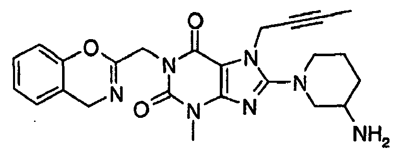 |
| (10) | 1-[(4,4-Dimethyl-4*H*-benzo[*e*][1,3]oxazin-2-yl)methyl]-3-methyl-7-(2-butin-1-yl)-8-(3-amino-piperidin-1-yl)-xanthin | 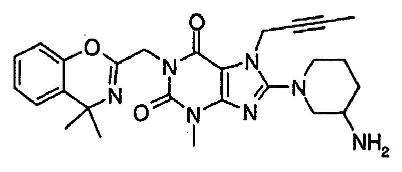 |
| (11) | 1-[(4-Oxo-4*H*-benzo[*e*][1,3]oxazin-2-yl)methyl]-3-methyl-7-(2-butin-1-yl)-8-(3-amino-piperidin-1-yl)-xanthin | 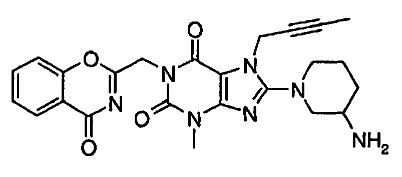 |
| (12) | 1-[(4*H*-Benzo[*d*][1,3]oxazin-2-yl)methyl]-3-methyl-7-(2-butin-1-yl)-8-(3-amino-piperidin-1-yl)-xanthin | 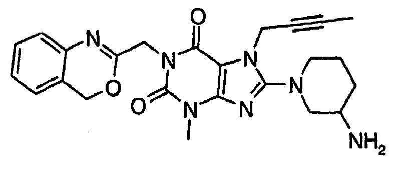 |
| (13) | 1-[(4,4-Dimethy)-4H-benzo[*d*][1,3]oxazin-2-yl)methyl]-3-methyl-7-(2-butin-1-yl)-8-(3-amino-piperidin-1-yl)-xanthin | 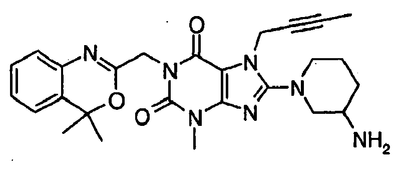 |
| (14) | 1-[(4-Oxo-4*H*-benzo[*d*][1,3]oxazin-2-yl)methyl]-3-methyl-7-(2-butin-1-yl)-8-(3-amino-piperidin-1-yl)-xanthin | 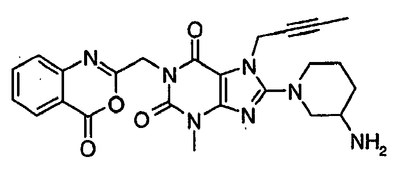 |
| (15) | 1-[(2*H*-Benzo[1,4]oxazin-3-yl)methyl]-3-methyl-7-(2-butin-1-yl)-8-(3-amino-piperidin-1-yl)-xanthin | 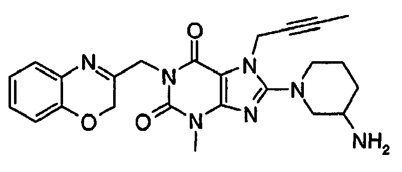 |
| (16) | 1-[(2-Oxo-2*H*-benzo[1,4]oxazin-3-yl)methyl]-3-methyl-7-(2-butin-1-yl)-8-(3-amino-piperidin-1-yl)-xanthin | 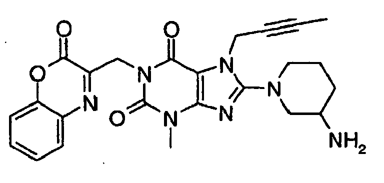 |
| (17) | 1-[(2,2-Dimethyl-2*H*-benzo[1,4]oxazin-3-yl)methyl]-3-methyl-7-(2-butin-1-yl)-8-(3-amino-piperidin-1-yl)-xanthin | 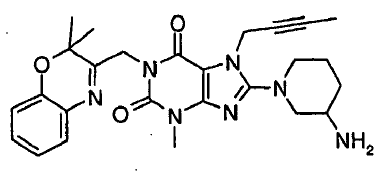 |
| (18) | 1-[4*H*-Benzo[e][1,3]thiazin-2-yl)methyl]-3-methyl-7-(2-butin-1-yl)-8-(3-amino-piperidin-1-yl)-xanthin | 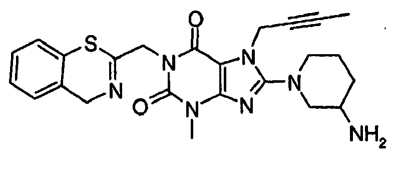 |
| (19) | 1-[4,4-Dimethyl-4*H*-benzo[e][1,3]thiazin-2-yl)methyl]-3-methyl-7-(2-butin-1-yl)-8-(3-amino-piperidin-1-yl)-xanthin | 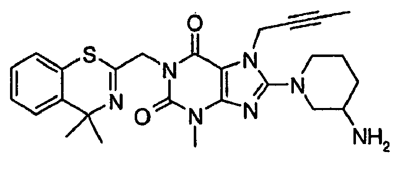 |
| (20) | 1-[4-Oxo-4*H*-benzo[*e*][1,3]thiazin-2-yl)methyl]-3-methyl-7-(2-butin-1-yl)-8-(3-amino-piperidin-1-yl)-xanthin | 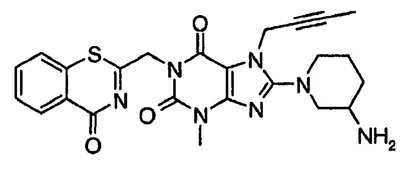 |
| (21) | 1-[(4*H*-Benzo[*d*][1,3]thiazin-2-yl)methyl]-3-methyl-7-(2-butin-1-yl)-8-(3-amino-piperidin-1-yl)-xanthin | 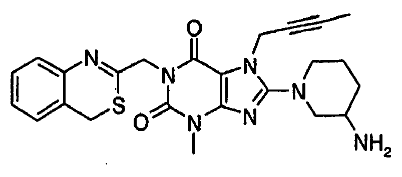 |
| (22) | 1-[(2*H*-Benzo[1,4]thiazin-3-yl)methyl]-3-methyl-7-(2-butin-1-yl)-8-(3-amino-piperidin-1-yl)-xanthin | 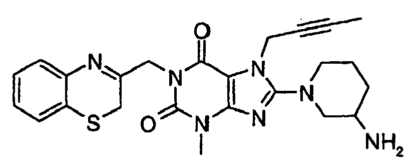 |
| (23) | 1-[(2-Oxo-2*H*-benzo[e][1,3]oxazin-4-yl)methyl]-3-methyl-7-(2-butin-1-yl)-8-(3-amino-piperidin-1-yl)-xanthin | 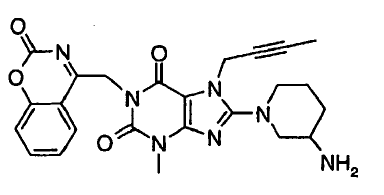 |
| (24) | 1-[(2,3-Dihydro-1*H*-benzo[e][1,4]diazepin-5-yl)methyl]-3-methyl-7-(2-butin-1-yl)-8-(3-amino-piperidin-1-yl)-xanthin | 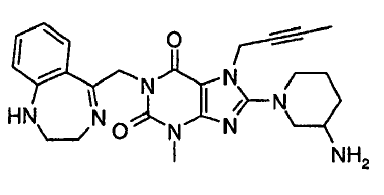 |
| (25) | 1-[(1-Methyl-2,3-dihydro-1*H*-benzo[*e*][1,4]diazepin-5-yl)methyl]-3-methyl-7-(2-butin-1-yl)-8-(3-amino-piperidin-1-yl)-xanthin | 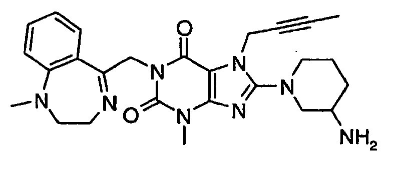 |
| (26) | 1-[(1-Methyl-2-oxo-2,3-dihydro-1*H*-benzo[e][1,4]diazepin-5-yl)methyl]-3-methyl-7-(2-butin-1-yl)-8-(3-amino-piperidin-1-yl)-xanthin | 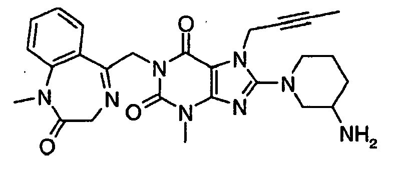 |
| (27) | 1-[(4-Oxo-4,5-dihydro-3*H*-benzo[*b*][1,4]diazepin-2-yl)methyl]-3-methyl-7-(2-butin-1-yl)-8-(3-amino-piperidin-1-yl)-xanthin | 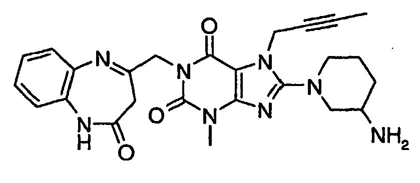 |
| (28) | 1-[(5-Methyl-4-oxo-4,5-dihydro-3*H*-benzo[*b*][1,4]diazepin-2-yl)methyl]-3-methyl-7-(2-butin-1-yl)-8-(3-amino-piperidin-1-yl)-xanthin | 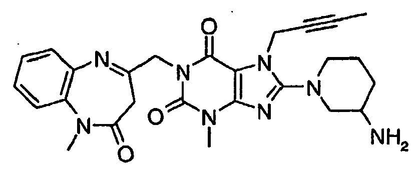 |
| (29) | 1-[5-Oxo-4,5-dihydro-3*H*-benzo[*e*][1,4]diazepin-2-yl)methyl]-3-methyl-7-(2-butin-1-yl)-8-(3-amino-piperidin-1-yl)-xanthin | 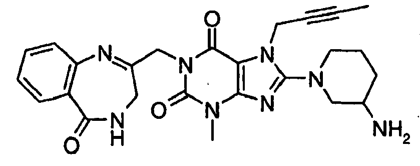 |
| (30) | 1-[4-Methyl-5-oxo-4,5-dihydro-3*H*-benzo[*e*][1,4]diazepin-2-yl)methyl]-3-methyl-7-(2-butin-1-yl)-8-(3-amino-piperidin-1-yl)-xanthin | 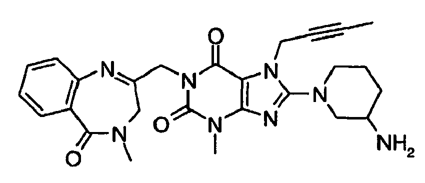 |
| (31) | 1-[(3,3-Dimethyl-2,3-dihydro-benzo[*f*][1,4]oxazepin-5-yl)methyl]-3-methyl-7-(2-butin-1-yl)-8-(3-amino-piperidin-1-yl)-xanthin | 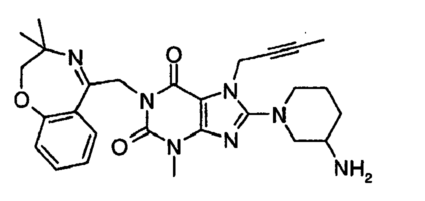 |
| (32) | 1-[(2,2-Dimethyl-2,3-dihydro-benzo[*f*][1,4]oxazepirl-5-yl)methyl]-3-methyl-7-(2-butin-1-yl)-8-(3-amino-piperidin-1-yl)-xanthin | 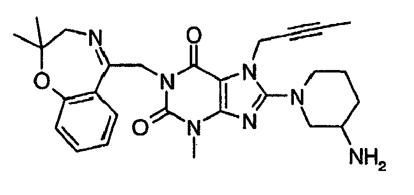 |
| (33) | 1-[(2,3-Dihydro-benzo[*b*][1,4]oxazepin-4-yl)methyl]-3-methyl-7-(2-butin-1-yl)-8-(3-amino-piperidin-1-yl)-xanthin | 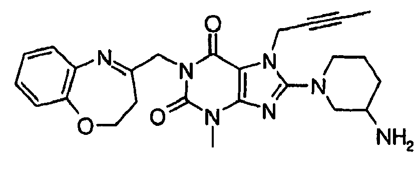 |
| (34) | 1-[(8,6-Dimethyl-2,3-dihydro-benzo[*b*][1,4]oxazepin-4-yl)methyl]-3-methyl-7-(2-butin-1-yl)-8-(3-amino-piperidin-1-yl)-xanthin | 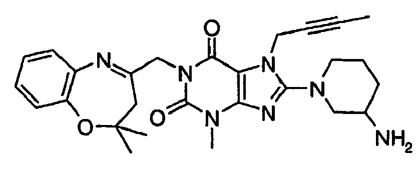 |
| (35) | 1-[(2,3-Dihydro-benzo[*b*][1,4]thiazepin-4-yl)methyl]-3-methyl-7-(2-butin-1-yl)-8-(3-amino-piperidin-1-yl)-xanthin | 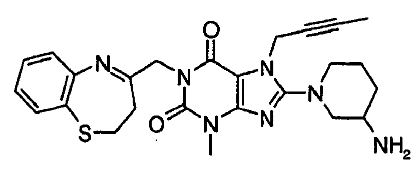 |
| (36) | 1-[(2,2-Dimethyl-2,3-dihydro-benzo[*b*][1,4]thiazepin-4-yl)methyl]-3-methyl-7-(2-butin-1-yl)-8-(3-amino-piperidin-1-yl)-xanthin | 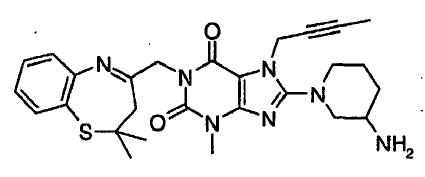 |
| (37) | 1-[(2,3-Dihydro-benzo[*f*][1,4]thiazepin-5-yl)methyl]-3-methyl-7-(2-butin-1-yl)-8-(3-amino-piperidin-1-yl)-xanthin | 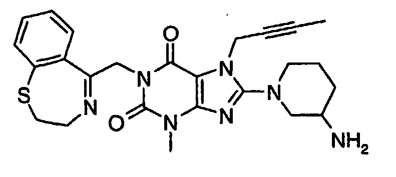 |
| (38) | 1-[(5-Oxo-4,5-dihydro-benzo[*f*][1,3,4]oxadiazepin-2-yl)methyl]-3-methyl-7-(2-butin-1-yl)-8-(3-amino-piperidin-1-yl)-xanthin | 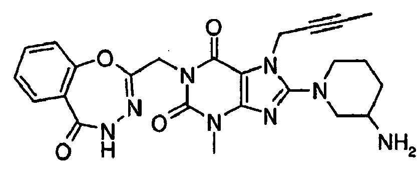 |
| (39) | 1-[(11*H*-Dibenzo[b,e]azepin-6-yl)methyl]-3-ethyl-7-(2-butin-1-yl)-8-(3-amino-piperidin-1-yl)-xanthin | 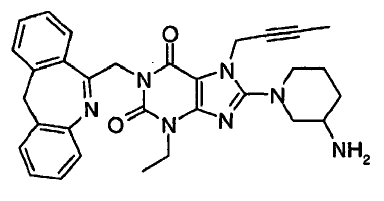 |
| (40) | 1-[(11*H*-Dibenzo[*b,e*]azepin-6-yl)methyl]-3-isopropyl-7-(2-butin-1-yl)-8-(3-amino-piperidin-1-yl)-xanthin | 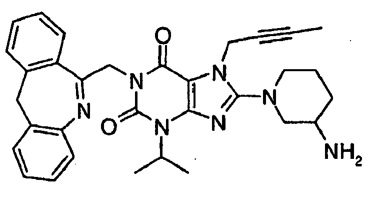 |
| (41) | 1-[(11-Oxo-11*H*-dibenzo[*b,e*]azepin-6-yl)methyl]-3-methyl-7-(2-butin-1-yl)-8-(3-amino-piperidin-1-yl)-xanthin | 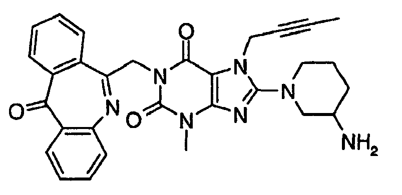 |
| (42) | 1-[(11*H*-Benzo[e]pyrido[3,2-b]azepin-6-yl)methyl]-3-methyl-7-(2-butin-1-yl)-8-(3-amino-piperidin-1-yl)-xanthin | 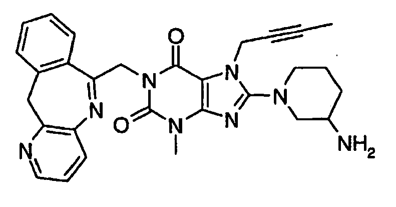 |
| (43) | 1-[(5-Methyl-5H dibenzo[b,e][1,4]diazepin-11-yl)methyl]-3-methyl-7-(2-butin-1-yl)-8-(3-amino-piperidin-1-yl)-xanthin | 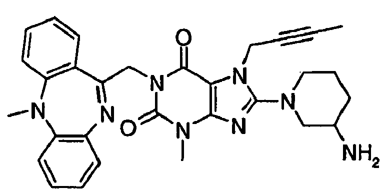 |
| (44) | 1-[(Dibenzo[*b,f*][1,4]thiazepin-11-yl)methyl]-3-methyl-7-(2-butin-1-yl)-8-(3-amino-piperidin-1-yl)-xanthin | 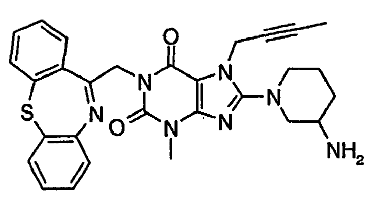 |
| (45) | 1-[(5-Oxo-dibenzo[*b,f*][1,4]thiazepin-11-yl)methyl]-3-methyl-7-(2-butin-1-yl)-8-(3-amino-piperidin-1-yl)-xanthin | 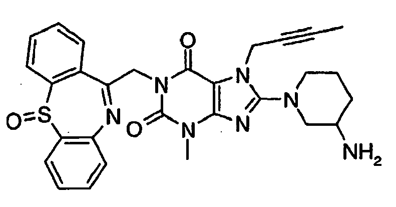 |
| (46) | 1-[(5,5-Dioxo-dibenzo[*b,f*][1,4]thiazepin-11-yl)methyl]-3-methyl-7-(2-butin-1-yl)-8-(3-amino-piperidin-1-yl)-xanthin | 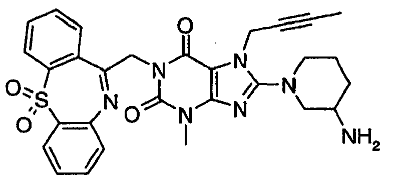 |
| (47) | 1-[(5*H*-Dibenzo[*a,d*]cyclohepten-10-yl)methyl]-3-methyl-7-(2-butin-1-yl)-8-(3-amino-piperidin-1-yl)-xanthin | 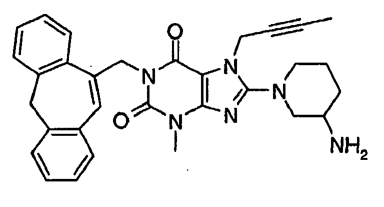 |
| (48) | 1-[(5-Methyl-5*H*-dibenzo[*b,f*]azepin-10-yl)methyl]-3-methyl-7-(2-butin-1-yl)-8-(3-amino-piperidin-1-yl)-xanthin | 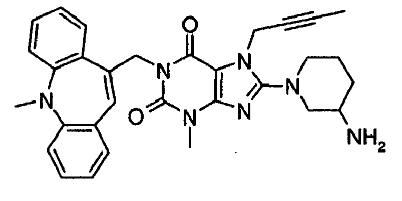 |
| (49) | 1-[(Phenanthridin-6-yl)methyl]-3-methyl-7-(3-methyl-2-buten-1-yl)-8-(3-amino-piperidin-1-yl)-xanthin | 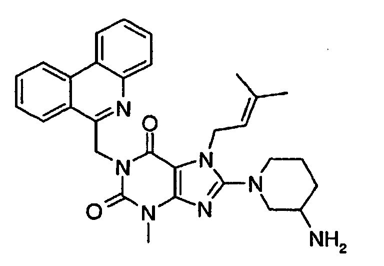 |
| (50) | 1-[(Phenanthridin-6-yl)methyl]-3-methyl-7-((E)-2-buten-1-yl)-8-(3-amino-piperidin-1-yl)-xanthin | 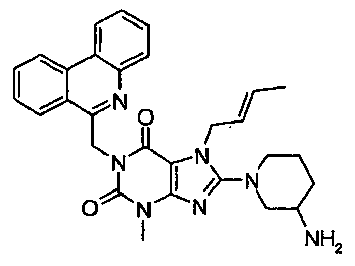 |
| (51) | 1-[(Phenanthridin-6-yl)methyl]-3-methyl-7-((Z)-2-buten-1-yl)-8-(3-amino-piperidin-1-yl)-xanthin | 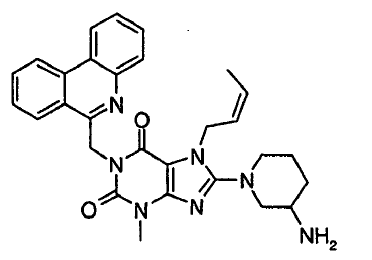 |
| (52) | 1-[(Phenanthridin-6-yl)methyl]-3-methyl-7-[(1-cyclopenten-1-yl)methyl]-8-(3-amino-piperidin-1-yl)-xanthin | 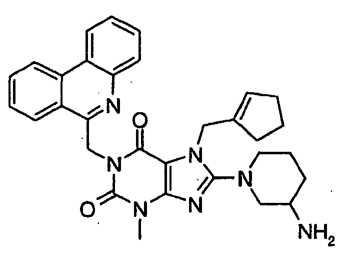 |
| (53) | 1-[(Benzo[c][1,5]naphthyridin-6-yl)methyl]-3-methyl-7-(2-butin-1-yl)-8-(3-amino-piperidin-1-yl)-xanthin | 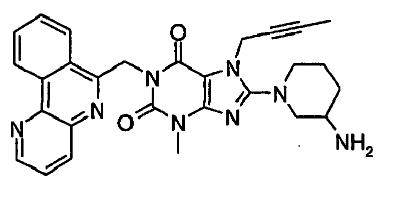 |
| (54) | 1-[(5*H*-Dibenzo[*d,f*][1,3]diazepin-6-yl)methyl]-3-methyl-7-(2-butin-1-yl)-8-(3-amino-piperidin-1-yl)-xanthin | 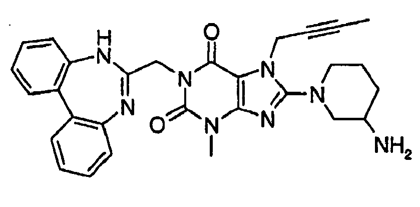 |
| (55) | 1-[(5*H*-Benzo[e]pyrrolo[1,2-a][1,4]diazepin-11-yl)methyl]-3-methyl-7-(2-butin-1-yl)-8-(3-amino-piperidin-1-yl)-xanthin | 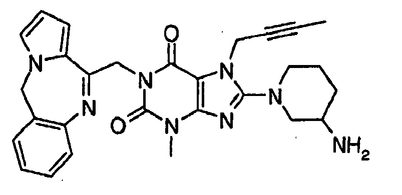 |
| (56) | 1-[(Thieno[3,2-b][1,4]benzoxazepin-9-yl)methyl]-3-methyl-7-(2-butin-1-yl)-8-(3-amino-piperidin-1-yl)-xanthin | 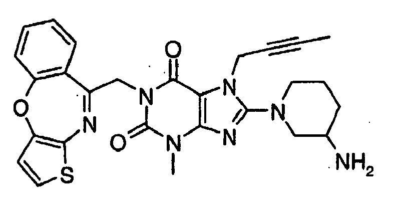 |
| (57) | 1-[(3,4-Dihydro-chinazolin-2-yl)methyl]-3-phenyl-7-(2-butin-1-yl)-8-(3-amino-piperidin-1-yl)-xanthin | 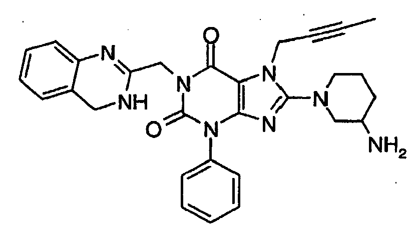 |
| (58) | 1-[(3,4-Dihydro-isochinolin-1-yl)methyl]-3-phenyl-7-(2-butin-1-yl)-8-(3-amino-piperidin-1-yl)-xanthin | 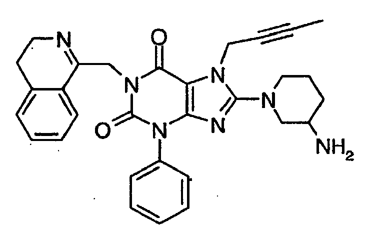 |
| (59) | 1-[(2,3-Dihydro-benzo[f][1,4]oxazepin-5-yl)methyl]-3-phenyl-7-(2-butin-1-yl)-8-(3-amino-piperidin-1-yl)-xanthin | 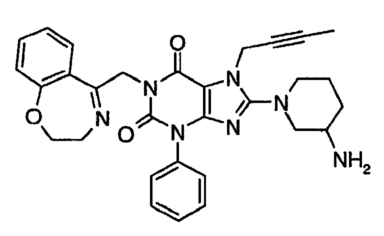 |
| (60) | 1-[(2.Oxo-2,3-dihydro-1H-benzo[*e*][1,4]diazepin-5-yl)methyl]-3-phenyl-7-(2-butin-1-yl)-8-(3-amino-piperidin-1-yl)-xanthin | 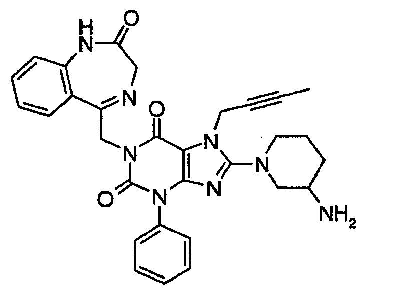 |

### Beispiel 4

### Dragees mit 75 mg Wirksubstanz

### 1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1.5 mg |
| | 230,0 mg |

### Herstellung

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.
Kerngewicht: 230 mg
Stempel: 9 mm, gewölbt
Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 5

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2.0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 6

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1.0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden

### Tabletten gepreßt.

| | |
|---|---|
| Tablettengewicht: | 300 mg |
| Stempel: | 10 mm, flach |

### Beispiel 7

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.
Kapseifüllung: ca. 320 mg
Kapselhülle: Hartgelatine-Kapsel Größe 1.

### Beispiel 8

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 9

### Suspension mit 50 mg Wirksubstanz

100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 10

### Ampullen mit 10 mg Wirksubstanz

Zusammensetzung:

| | |
|---|---|
| Wirkstoff | 10,0 mg |
| 0,01 n Salzsäure s.q. Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 11

### Ampullen mit 50 mg Wirksubstanz

Zusammensetzung:

| | |
|---|---|
| Wirkstoff | 50,0 mg |
| 0,01 n Salzsäure s.q. Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 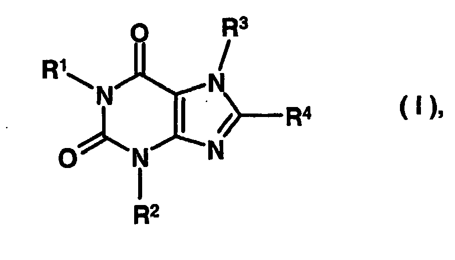 in der
R¹ eine durch eine Gruppe Rₐ substituierte C₁₋₃-Alkylgruppe, wobei
Rₐ eine 1,4-Dihydro-chinazolinyl- oder 3,4-Dihydro-chinazolinylgruppe, in der jeweils im Benzoteil
eine bis drei Methingruppen durch Stickstoffatome ersetzt sein können,
eine 3,4-Dihydro-isochinolinyl-, 1*H*-Benzo[*d*][1,2]oxazinyl-, 4*H*-Benzo[e][1,3]oxazinyl-, 4*H*-Benzo[*d*][1,3]oxazinyl- oder 2*H*-Benzo[1,4]oxazinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 4*H*-Benzo[*e*][1,3]thiazinyl-, 4*H*-Benzo[*d*][1,3]thiazinyl- oder 2*H-*Benzo[1,4]thiazinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann sowie ein Schwefelatom durch eine Sulfinyl- oder Sulfonylgruppe ersetzt sein kann,
eine 2-Oxo-2*H*-benzo[*e*][1,3]oxazinyl- oder 2,2-Dioxo-1*H*-benzo[*c*][1,2]thiazinylgruppe, in der jeweils im Benzoteil
eine bis drei Methingruppen durch Stickstoffatome ersetzt sein können,
eine 2,3-Dihydro-1*H-*benzo[*e*][1,4]diazepinyl-, 4,5-Dihydro-3*H*-benzo[*b*]-[1,4]diazepinyl- oder 5-Oxo-4,5-dihydro-3*H*-benzo[*e*][1,4]diazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 2,3-Dihydro-benzo[*f*][1,4]oxazepinyl- oder 2,3-Dihydro-benzo[b][1,4]oxazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann,
eine 2,3-Dihydro-benzo[*f*][1,4]thiazepinyl- oder 2,3-Dihydrobenzo[*b*][1,4]thiazepinylgruppe, in der jeweils
im Benzoteil eine bis drei Methingruppen durch Stickstoffatome ersetzt sein können und im Heterocyclylteil eine Methylengruppe durch eine Carbonylgruppe ersetzt sein kann und ein Schwefelatom durch eine Sulfinyl- oder Sulfonylgruppe ersetzt sein kann,
eine 5-Oxo-4,5-dihydro-benzo[*f*][1,3,4]oxadiazepinylgruppe, in der im Benzoteil
eine bis drei Methingruppen durch Stickstoffatome ersetzt sein können,
eine 11*H*-Dibenzo[*b,e*]azepinyl- oder 5H-Dibenzo[*a,d*]cycloheptenylgruppe, in der jeweils
im Benzoteil eine bis drei Methingruppen durch Stickstoffatome ersetzt sein können und die Methylengruppe im Heterocyclylteil durch ein Sauerstoff- oder Schwefelatom, eine Carbonyl-, Sulfinyl-, Sulfonyl- oder eine durch Rₓ substituierte Iminogruppe ersetzt sein kann, wobei
Rₓ ein Wasserstoffatom oder eine C₁₋₄-Alkyl-, C₂₋₄-Alkenyl-, C₂-₄-Alkinyl-, C₃₋₆-Cycloalkyl-, C₃₋₆-cycloalkyl-C₁₋₃-alkyl-, Aryl-, Aryl-C₁₋₃-alkyl-, Hydroxy-C₂₋₄-alkyl-, C₁₋₃-Alkyloxy-C₂₋₄-alkyl-, C₃₋₆-Cycloalkyloxy-C₂₋₄-alkyl-, Amino-C₂₋₄-alkyl-, C₁₋₃-Alkylamino-C₂₋₄-alkyl, Di-(C₁₋₃-alkyl)-amino-C₂₋₄-alkyl-, C₁₋₃-Alkyl-carbonyl-, C₁₋₃-Alkyloxy-carbonyl-, C₁₋₃-Alkyloxy-carbonyl-C₁₋₃-alkyl-, Aryl-carbonyl-, C₁₋₃-Alkyl-sulfonyl- oder Aryl-sulfonylgruppe darstellt,
eine Phenanthridinyl-, 1,2,3,4-Tetrahydro-phenanthridinyl-, Benzo[*f*]chinoxalinyl-, 5*H*-Dibenzo[*d,f*][1,3]diazepinyl-, 5H-Benzo[*e*]pyrrolo[1,2-a][1,4]diazepinyl-, Thieno[3,2-b][1,4]benzoxazepinyl- oder eine 3-Oxo-2,3-dihydro-isoindol-1-ylidengruppe, in der jeweils
im Benzoteil eine bis drei Methingruppen durch Stickstoffatome ersetzt sein können,
eine Benzo[1,2,5]oxadiazolyl-, Dibenzofuranyl-, Indolizinyl-, 1*H*-Perimidinyl-, gruppe,
eine Pyrazolo[1,5-c]chinzolinylgruppe oder
eine Imidazo[2,1-a]isochinolinyl- oder Imidazo[1,2-a]isochinolinylgruppe bedeutet, wobei die vorstehend erwähnten Reste Rₐ durch die Gruppen R¹⁰ bis R¹³ substituiert sein können und zusätzlich durch eine C₁₋₃-Alkylgruppe substituiert sein können und
R¹⁰ ein Wasserstoffatom,
ein Fluor-, Chlor-, Brom- oder lodatom,
eine C₁₋₄-Alkyl-, Hydroxy-, oder C₁₋₄-Alkyloxygruppe,
eine Nitro-, Amino-, C₁₋₃-Alkylamino-, Di-(C₁₋₃-alkyl)amino-, Cyan-C₁₋₃-alkylamino-, [N-(Cyan-C₁₋₃-alkyl)-N-C₁₋₃-alkyl-amino]-, C₁₋₃-Alkyloxycarbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
eine C₁₋₃-Alkyl-carbonylamino-, Arylcarbonylamino-, Aryl-C₁₋₃-alkylcarbonylamino-, C₁₋₃-Alkyloxy-carbonylamino-, Aminocarbonylamino-, C₁₋₃-Alkyl-aminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkylaminosulfonylamino-, Di-(C₁₋₃-alkyl)amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thlocarbonylamino-, (C₁₋₃-Alkyloxy-carbonylamino)carbonylamino-, Arylsulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkylsulfonylamino-, N-(C₁₋₃-Alkyl)-arylsulfonylamino-, oder N-(C₁₋₃-Alkyl)-aryl-C₁₋₃-alkyl-sulfonylaminogruppe,
eine 2-Oxo-imidazolidin-1-yl-, 2,4-Dioxo-imidazolidin-1-yl-, 2,5-Dioxoimidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1-yl-carbonylgruppe,
eine C₁₋₃-Alkyl-carbonyl- oder eine Arylcarbonylgruppe,
eine carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkylaminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe, eine Hydroxy-C₁₋₃-alkyloxy-, C₁₋₃-Alkyloxy-C₁₋₃-alkyloxy-, C₁₋₃-Alkylsulfanyl-C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl- oder Trifluormethylsulfonylgruppe,
eine Sulfo-, Aminosulfonyl-, C₁₋₃-Alkyl-aminosulfonyl-, Di-(C₁₋₃-alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonylgruppe,
eine durch 1 bis 3 Fluoratome substituierte Methyl- oder Methoxygruppe,
eine durch 1 bis 5 Fluoratome substituierte Ethyl- oder Ethoxygruppe, eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, oder C₁₋₃-Alkyloxygruppe oder eine Cyangruppe, oder
R¹¹ zusammen mit R¹², sofern diese an benachbarte Kohlenstoffatome gebunden sind, auch eine Methylendioxy-, Difluormethylendioxy-, Ethylendioxy- oder eine geradkettige C₃₋₅-Alkylengruppe
und
R¹³ ein Wasserstoffatom, ein Fluor-, Chlor- oder Bromatom, eine Trifluormethyl-, C₁₋₃-Alkyl- oder C₁₋₃-Alkyloxygruppe bedeuten,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonyl-C₁₋₂-alkylgruppe,
eine Heteroaryl-C₁₋₃-alkylgruppe,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆-Cycloalkyl-carbonyl-C₁₋₂-alkyl-Gruppe,
eine Aryl-A-C₁₋₃-alkylgruppe, wobei A eine Sauerstoff- oder Schwefelatom, eine Imino-, C₁₋₃-Alkylimino-, Sulfinyl- oder Sulfonylgruppe bedeutet,
eine durch eine Gruppe R_{b} substituierte C₁₋₄-Alkylgruppe, wobei
R_{b} eine Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
oder eine durch eine Gruppe R_{c} substituierte C₂₋₄-Alkylgruppe, wobei
R_{c} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 3-Stellung des Xanthingerüstes isoliert ist,
R³ eine C₃₋₈-Alkylgruppe,
eine durch eine Gruppe R_{d} substituierte C₁₋₃-Alkylgruppe, wobei
R_{d} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyanthiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycioalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminögruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Ccyloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piporidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder Iodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

2. Verbindungen der allgemeinen Formel I gemäß Anspruch 1, in denen R¹ eine durch eine Gruppe Rₐ substituierte Methylgruppe, wobei
Rₐ eine 1,4-Dihydro-chinazolinyl- oder 3,4-Dihydro-chinazolinylgruppe,
eine 3,4-Dihydro-isochinolinylgruppe,
eine 1*H*-Benzo[*d*][1,2]oxazinyl- oder 1-Oxo-1*H*-benzo[*d*][1,2]oxazinylgruppe, eine 4*H*-Benzo[*e*][1,3]oxazinyl- oder 4-Oxo-4H-benzo[*e*][1,3]oxazinylgruppe,
eine 4*H*-Benzo[*d*][1,3]oxazinyl- oder 4-Oxo-4*H*-benzo[*d*][1,3]oxazinylgruppe, 2*H*-Benzo[1,4]oxazinyl- oder 2-Oxo-2*H*-benzo[1,4]oxazinylgruppe,
eine 4*H*-Benzo[*e*][1,3]thiazinyl- oder 4-Oxo-4*H*-benzo[*e*][1,3]thiazinylgruppe, eine 4*H*-Benzo[*d*][1,3]thiazinyl- oder 2*H*-Benzo[1,4]thiazinylgruppe,
eine 2-Oxo-2*H*-benzo[*e*][1,3]oxazinyl- oder 2,2-Dioxo-1*H-*benzo[*c*][1,2]thiazinylgruppe,
eine 2,3-Dihydro-1*H*-benzo[*e*][1,4]diazepinyl- oder 2-Oxo-2,3-dihydro-1*H-*benzo[*e*][1,4]diazepinylgruppe,
eine 4,5-Dihydro-3*H*-benzo[*b*][1,4]diazepinyl- oder 4-Oxo-4,5-dihydro-3*H-*benzo[*b*][1,4]diazepinylgruppe,
eine 5-Oxo-4,5-dihydro-3*H*-benzo[e][1,4]diazepinylgruppe,
eine 2,3-Dihydro-benzo[*f*][1,4]oxazepinyl- oder 2,3-Dihydrobenzo[*b*][1,4]oxazepinylgruppe,
eine 2,3-Dihydro-benzo[*f*][1,4]thiazepinyl- 2,3-Dihydrobenzo[*b*][1,4]thiazepinylgruppe,
eine 5-Oxo-4,5-dihydro-benzo[*f*][1,3,4]oxadiazepinylgruppe,
eine 11*H*-Dibenzo[*b,e*]azepinyl- oder 11-Oxo-11*H*-dibenzo[*b,e*]azepinylgruppe, eine 11*H*-Benzo[*e*]pyrido[3,2-b]azepinylgruppe,
eine 5*H*-Dibenzo[b,e][1,4]diazepinyl- oder Dibenzo[*b,f*][1,4]oxazepinylgruppe,
eine Dibenzo[*b,f*][1,4]thiazepinyl-, 5-Oxo-dibenzo[*b,f*][1,4]thiazepinyl- oder 5,5-Dioxo-dibenzo[*b,f*][1,4]thiazepinylgruppe,
5H-Dibenzo[*a,d*]cycloheptenyl- oder 5*H*-Dibenzo[*b,f*]azepinylgruppe,
eine Phenanthridinyl-, Benzo[*c*][1,5]naphthyridinyl-, Benzo[*h*][1,6]naphthyridinyl-, Benzo[*c*][1,8]naphthyridinyl- oder 1,2,3,4-Tetrahydro-phenanthridinylgruppe,
eine Benzo[*f*]chinoxalinylgruppe,
eine 5*H*-Dibenzo[*d,f*][1,3]diazepinyl-, 5H-Benzo[*e*]pyrrolo[1,2-a][1,4]diazepinyl- oder Thieno[3,2-b][1,4]benzoxazepinylgruppe,
eine 3-Oxo-2,3-dihydro-isoindol-1-ylidengruppe,
eine Benzo[1,2,5]oxadiazolylgruppe,
eine Dibenzofuranylgruppe,
eine Indolizinylgruppe,
eine 1*H*-Perimidinylgruppe,
eine Pyrazolo[1,5-c]chinzolinylgruppe oder
eine Imidazo[2,1-a]isochinolinyl- oder Imidazo[1,2-a]isochinolinylgruppe bedeutet, wobei die Benzogruppen der vorstehend erwähnten Reste Rₐ durch die Gruppen R¹⁰ bis R¹² substituiert sind und die Alkyleneinheiten der vorstehend erwähnten Reste Rₐ durch eine oder zwei C₁₋₃-Alkyl- oder C₁₋₃-Alkyloxy-carbonylgruppen, wobei die Reste gleich oder verschieden sein können, oder durch eine Trifluormethylgruppe substituiert sein können und die Iminogruppen der vorstehend erwähnten Reste Rₐ durch eine C₁₋₃-Alkylgruppe substituiert sein können und
R¹⁰ ein Wasserstoffatom,
ein Fluor-, Chlor-, Brom- oder lodatom,
eine C₁₋₃-Alkyl- oder Cyclopropylgruppe,
eine Hydroxy-, C₁₋₃-Alkyloxy- oder Cyclopropyloxygruppe,
eine Nitro-, Amino-, C₁₋₃-Alkylamino- oder Di-(C₁₋₃-alkyl)aminogruppe, eine C₁₋₃-Alkyl-carbonylamino- oder C₁₋₃-Alkyl-sulfonylaminogruppe, eine Cyan-, Carboxy-, C₁₋₃-Alkyloxy-carbonyl-, Aminocarbonyl-, C₁₋₃-Alkyl-aminocarbonyl- oder Di-(C₁₋₃-alkyl)-aminocarbonylgruppe,
eine Mercapto-, C₁₋₃-Alkylsulfanyl-, C₁₋₃-Alkysulfinyl-,oder C₁₋₃-Alkylsulfonyl- oder Aminosulfonylgruppe oder
eine Difluormethyl-, Trifluormethyl-, Difluormethoxy- oder Trifluormethoxygruppe und
R¹¹ und R¹², die gleich oder verschieden sein können, jeweils ein Wasserstoffatom, ein Fluor-, Chlor- oder Bromatom, eine Methyl-, Trifluormethyl- oder Methoxygruppe bedeuten,
R² ein Wasserstoffatom,
eine C₁₋₃-Alkylgruppe,
eine C₃₋₆Cycloalkylgruppe oder
eine gegebenenfalls durch ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cydopropyl., Ethenyl-, Ethinyl-, Hydroxy-, C₁₋₃-Alkyloxy-, Difluormethoxy- oder Trifluormethoxygruppe mono- oder disubstituierte Phenylgruppe, wobei die Substituenten gleich oder verschieden sein können,
R³ eine 2-Buten-1-yl- oder 3-Methyl-2-buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
wobei, soweit nichts anderes erwähnt wurde, die vorstehend erwähnten Alkylgruppen geradkettig oder verzweigt sein könnnen,
deren Tautomere, Enantiomere, Diastereomere, deren Gemische und deren Salze.

3. Verbindungen der allgemeinen Formel I gemäß Anspruch 2, in denen R¹ eine durch eine Gruppe Rₐ substituierte Methylgruppe, wobei
Rₐ eine 1,4-Dihydro-chinazolin-2-yl- oder 3,4-Dihydro-chinazolin-2-ylgruppe, eine 3,4-Dihydro-isochinolin-1-ylgruppe,
eine 1*H*-Benzo[*d*][1,2]oxazin-4-yl- oder 1-Oxo-1*H*-benzo[*d*][1,2]oxazin-4-ylgruppe,
eine 4*H*-Benzo[*e*][1,3]oxazin-2-yl- oder 4-Oxo-4H-benzo[*e*][1,3]oxazin-2-ylgruppe,
eine 4*H*-Benzo[*d*][1,3]oxazin-2-yl- oder 4-Oxo-4*H*-benzo[*d*][1,3]oxazin-2-ylgruppe,
2*H*-Benzo[1,4]oxazin-3-yl- oder 2-Oxo-2*H*-benzo[1,4]oxazin-3-ylgruppe,
eine 4*H*-Benzo[*e*][1,3]thiazin-2-yl- oder 4-Oxo-4*H*-benzo[*e*][1,3]thiazin-2-ylgruppe,
eine 4*H*-Benzo[*a*][1,3]thiazin-2-yl- oder 2*H*-Benzo[1,4]thiazin-3-ylgruppe, eine 2-Oxo-2*H*-benzo[*e*][1,3]oxazin-4-yl- oder 2,2-Dioxo-1*H*-benzo[*c*][1,2]thiazin-4-ylgruppe,
eine 2,3-Dihydro-1*H*-benzo[*e*][1,4]diazepin-5-yl- oder 2-Oxo-2,3-dihydro-1*H-*benzo[*e*][1,4]diazepin-5-ylgruppe,
eine 4,5-Dihydro-3*H*-benzo[*b*][1,4]diazepin-2-yl- oder 4-Oxo-4,5-dihydro-3*H-*benzo[*b*][1,4]diazepin-2-ylgruppe,
eine 5-Oxo-4,5-dihydro-3*H*-benzo[*e*][1,4]diazepin-2-ylgruppe,
eine 2,3-Dihydro-benzo[*f*][1,4]oxazepin-5-yl- oder 2,3-Dihydro-benzo[*b*]-[1,4]oxazepin-4-ylgruppe,
eine 2,3-Dihydro-benzo[*f*][1,4]thiazepin-5-yl- oder 2,3-Dihydro-benzo[*b*]-[1,4]thiazepin-4-ylgruppe,
eine 5-Oxo-4,5-dihydro-benzo[*f*][1,3,4]oxadiazepin-2-ylgruppe,
eine 11*H*-Dibenzo[*b,e*]azepin-6-yl- oder 11-Oxo-11*H*-dibenzo[*b,e*]azepin-6-ylgruppe,
eine 11*H*-Benzo[*e*]pyrido[3,2-*b*]azepin-6-ylgruppe
eine 5*H*-Dibenzo[*b,e*][1,4]diazepin-11-yl- oder Dibenzo[*b,f*][1,4]oxazepin-11-ylgruppe,
eine Dibenzo[*b,f*][1,4]thiazepin-11-yl-, 5-Oxo-dibenzo[*b,f*][1,4]thiazepin-11-yl-oder 5,5-Dioxo-dibenzo[*b,f*][1,4]thiazepin-11-ylgruppe,
eine 5H-Dibenzo[*a,d*]cyclohepten-10-yl- oder 5*H*-Dibenzo[*b,f*]zepin-10-ylgruppe,
eine Phenanthridin-6-yl-, Benzo[*c*][1,5]naphthyridin-6-yl-, Benzo[*h*][1,6]naphthyridin-5-yl-, Benzo[*c*][1,8]naphthyridin-6-yl- oder 1,2,3,4-Tetrahydro-phenanthridin-6-ylgruppe,
eine Benzo[*f*]chinoxalin-6-ylgruppe,
eine 5*H*-Dibenzo[*d,f*][1,3]diazepin-6-yl-, 5H-Benzo[*e*]pyrrolo[1,2-*a*][1,4]diazepin-11-yl- oder Thieno[3,2-b][1,4]benzoxazepinyl-9-ylgruppe,
eine 3-Oxo-2,3-dihydro-isoindol-1-ylidengruppe,
eine Benzo[1,2,5]oxadiazol-5-ylgruppe,
eine Dibenzofuran-2-ylgruppe,
eine Indolizin-2-ylgruppe,
eine 1*H*-Perimidin-2-ylgruppe,
eine Pyrazolo[1,5-c]chinzolin-5-ylgruppe oder
eine Imidazo[2,1-a]isochinolin-2-yl- oder Imidazo[1,2-a]isochinolin-2-ylgruppe bedeutet, wobei die Benzogruppen der vorstehend erwähnten Reste Rₐ durch die Gruppen R¹⁰ bis R¹² substituiert sind und die Alkyleneinheiten der vorstehend erwähnten Reste Rₐ durch eine oder zwei Methyl- oder Methoxycarbonylgruppen, wobei die Reste gleich oder verschieden sein können, oder durch eine Trifluormethylgruppe substituiert sein können und die Iminogruppen der vorstehend erwähnten Reste Rₐ durch eine Methylgruppe substituiert sein können und
R¹⁰ ein Wasserstoffatom,
ein Fluor-, Chlor-, Brom- oder Iodatom,
eine Methyl- oder Ethylgruppe,
eine Hydroxy-, Methoxy- oder Ethoxygruppe oder
eine Difluormethyl-, Trifluormethyl-, Difluormethoxy-, oder Trifluormethoxygruppe und
R¹¹ und R¹², die gleich oder verschieden sein können, jeweils ein Wasserstoffatom, ein Fluor-, Chlor- oder Bromatom, eine Methyl-, Trifluormethyl- oder Methoxygruppe bedeuten,
R² ein Wasserstoffatom oder
eine Methyl-, Ethyl-, Propyl-, Isopropyl-, Phenyl- oder Cyclopropylgruppe,
R³ eine 2-Buten-1-yl- oder 3-Methyl-2-buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
deren Tautomere, Enantiomere, Diastereomere, deren Gemische und deren Salze.

4. Verbindungen der allgemeinen Formel I gemäß Anspruch 3, in denen R¹ eine 3-Methoxycarbonyl-3-methyl-3,4-dihydro-isochinolin-1-ylmethylgruppe, eine 1-Methyl-2,2-dioxo-1*H-*benzo[*c*][1,2]thiazin-4-ylmethylgruppe,
eine 2,3-Dihydro-benzo[*f*][1,4]oxazepin-5-ylmethylgruppe,
eine 2-Oxo-2,3-dihydro-1*H*-benzo[*e*][1,4]diazepin-5-ylmethylgruppe,
eine Phenanthridin-6-ylmethyl- oder 1,2,3,4-Tetrahydro-phenanthridin-6-ylmethylgruppe,
eine 11*H*-Dibenzo[*b,e*]azepin-6-ylmethylgruppe,
eine Dibenzo[*b,f*][1,4]oxazepin-11-ylmethylgruppe,
eine 3-Oxo-2,3-dihydro-isoindol-1-ylidenmethylgruppe,
eine 3-Trifluormethyl-3,4-dihydro-isochinolin-1-ylmethylgruppe,
eine 3,4-Dihydro-chinazolin-2-ylmethylgruppe,
eine 5-Methyl-5H-dibenzo[*b,e*][1,4]diazepin-11-ylmethylgruppe,
eine 8-Methyl-dibenzo[b,*f*][1,4]oxazepin-11-ylmethylgruppe,
eine Benzo[1,2,5]oxadiazol-5-ylmethylgruppe,
eine 8-Methyl-phenanthridin-6-ylmethylgruppe,
eine 1-Methyl-phenanthridin-6-ylmethylgruppe,
eine 4-Methyl-phenanthridin-6-ylmethylgruppe,
eine Benzo[*h*][1,6]naphthyridin-5-ylmethylgruppe,
eine Pyrazolo[1,5-*c*]chinzolin-5-ylgruppe,
eine Benzo[*c*][1,8]naphthyridin-6-ylmethylgruppe,
eine Benzo[*c*][1,5]naphthyridin-6-ylmethylgruppe,
eine 1*H-*Perimidin-2-ylmethylgruppe,
eine Benzo[*f*]chinoxalin-6-ylmethylgruppe oder
eine Imidazo[2,1-a]isochinolin-2-ylmethyl- oder Imidazo[1,2-a]isochinolin-2-ylmethylgruppe,
R² eine Methyl- oder Cyclopropylgruppe,
R³ eine 2-Buten-1-yl-, 3-Methyl-2-buten-1-yl- oder 2-Butin-1-ylgruppe
und
R⁴ eine (3-Amino-piperidin-1-yl)gruppe bedeuten,
deren Tautomere, Enantiomere, Diastereomere, deren Gemische und deren Salze.

5. Folgende Verbindungen der allgemeinen Formel I gemäß Anspruch 1:
(1) 1-[(1-Methyl-2,2-dioxo-1*H*-benzo[*c*][1,2]thiazin-4-yl)methyl]-3-methyl-7-(3-methyl-2-buten-1-yl)-8-(3-amino-piperidin-1-yl)-xanthin,
(2) 1-[(3-Methoxycarbonyl-3-methyl-3,4-dihydro-isochinolin-1-yl]methyl]-3-methyl-7-(2-butin-1-yl)-8-(3-amino-piperidin-1-yl)-xanthin,
(3) 1-[(2-Oxo-2,3-dihydro-1H-benzo[*e*][1,4]diazepin-5-yl)methyl]-3-methyl-7-((E)-2-buten-1-yl)-8-((*R*)-3-amino-piperidin-1-yl)-xanthin,
(4) 1-[(Phenanthridin-6-yl)methyl]-3-methyl-7-(2-butin-1-yl)-8-(3-amino-piperidin-1-yl)-xanthin,
(5) 1-[(1,2,3,4-Tetrahydro-phenanthridin-6-yl)methyl]-3-methyl-7-(2-butin-1-yl)-8-(3-amino-piperidin-1-yl)-xanthin,
(6) 1-[(11*H*-Dibenzo[*b,e*]azepin-6-yl)methyl]-3-methyl-7-(2-butin-1-yl)-8-(3-aminopiperidin-1-yl)-xanthin,
(7) 1-[(Dibenzo[*b,f*][1,4]oxazepin-11-yl)methyl]-3-methyl-7-(2-butin-1-yl)-8-(3-aminopiperidin-1-yl)-xanthin,
(8) 1-[(3-Trifluormethyl-3,4-dihydro-isochinolin-1-yl)methyl]-3-methyl-7-(2-butin-1-yl)-8-(3-amino-piperidin-1-yl)-xanthin,
(9) 1-[(Dibenzo[*b,f*][1,4]oxazepin-11-yl)methyl]-3-methyl-7-(2-butin-1-yl)-8-((*R*)-3-amino-piperidin-1-yl)-xanthin,
(10) 1-[(3,4-Dihydro-chinazolin-2-yl)methyl]-3-methyl-7-(2-butin-1-yl)-8-(3-aminopiperidin-1-yl)-xanthin,
(11) 1-[(5-Methyl-5H-dibenzo[*b,e*][1,4]diazepin-11-yl)methyl]-3-methyl-7-(2-butin-1-yl)-8-(3-amino-piperidin-1-yl)-xanthin,
(12) 1-[(8-Methyl-dibenzo[*b,f*][1,4]oxazepin-11-yl)methyl]-3-methyl-7-(2-butin-1-yl)-8-(3-amino-piperidin-1-yl)-xanthin,
(13) 1-[(Benzo[1,2,5]oxadiazol-5-yl)methyl]-3-methyl-7-(2-butin-1-yl)-8-(3-aminopiperidin-1-yl)-xanthin,
(14) 1-[(Phenanthridin-6-yl)methyl]-3-cyclopropyl-7-(2-butin-1-yl)-8-(3-aminopiperidin-1-yl)-xanthin,
(15) 1-[(8-Methyl-phenanthridin-6-yl)methyl]-3-methyl-7-(2-butin-1-yl)-8-(3-aminopiperidin-1-yl)-xanthin,
(16) 1-[(Phenanthridin-6-yl)methyl]-3-methy)-7-(2-butin-1-yl)-8-((*S*)-3-amino-piperidin-1-yl)-xanthin ,
(17) 1-[(Phenanthridin-6-yl)methyl]-3-cyclopropyl-7-(2-butin-1-yl)-8-((*R*)-3-aminopiperidin-1-yl)-xanthin,
(18) 1-[(1-Methyl-phenanthridin-6-yl)methyl]-3-methyl-7-(2-butin-1-yl)-8-((*R*)-3-aminopiperidin-1-yl)-xanthin,
(19) 1-[(4-Methyl-phenanthridin-6-yl)methyl]-3-methyl-7-(2-butin-1-yl)-8-((*R*)-3-aminopiperidin-1-yl)-xanthin,
(20) 1-[(Benzo[*h*][1,6]naphthyridin-5-yl)methyl]-3-methyl-7-(2-butin-1-yl)-8-(3-aminopiperidin-1-yl)-xanthin,
(21) 1-[(Pyrazolo[1,5-c]chinazolin-5-yl)methyl]-3-methyl-7-(2-butin-1-yl)-8-(3-aminopiperidin-1-yl)-xanthin,
(22) 1-[(Benzo[*c*][1,8]naphthyridin-6-yl)methyl]-3-methyl-7-(2-butin-1-yl)-8-(3-aminopiperidin-1-yl)-xanthin,
(23) 1-[(Benzo[*c*][1,5]naphthyridin-6-yl)methyl]-3-methyl-7-(2-butin-1-yl)-8-((*R*)-3-amino-piperidin-1-yl)-xanthin,
(24) 1-[(1*H*-Perimidin-2-yl)methyl]-3-methyl-7-(2-butin-1-yl)-8-((*R*)-3-amino-piperidin-1-yl)-xanthin,
(25) 1-[(Benzo[*f*]chinoxalin-6-yl)methyl]-3-methyl-7-(2-butin-1-yl)-8-((*R*)-3-aminopiperidin-1-yl)-xanthin,
(26) 1-[(Imidazo[2,1-a]isochinolin-2-yl)methyl]-3-methyl-7-(2-butin-1-yl)-8-(3-aminopiperidin-1-yl)-xanthin,
(27) 1-[(Imidazo[1,2-a]isochinolin-2-yl)methyl]-3-methyl-7-(2-butin-1-yl)-8-(3-aminopiperidin-1-yl)-xanthin,
(28) 1-[(Phenanthridin-6-yl)methyl]-3-methyl-7-(2-butin-1-yl)-8-((*R*)-3-amino-piperidin-1-yl)-xanthin;
(29) 1-[(2,3-Dihydro-benzo[*t*][1,4]oxazepin-5-yl)methyl]-3-methyl-7-(2-butin-1-yl)-8-(3-amino-piperidin-1-yl)-xanthin, und
(30) 1-[(3-Oxo-2,3-dihydro-isoindol-1-yliden)methyl]-3-methyl-7-(3-methyl-2-buten-1-yl)-8-(3-amino-piperidin-1-yl)-xanthin
sowie deren Salze.

6. Physiologisch verträgliche Salze der Verbindungen nach mindestens einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

9. Verfahren zur Herstellung eines Arzneimittels gemäß Anspruch 7, **dadurch gekennzeichnet, daß** auf nichtchemischen Weg eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der Verbindungen der allgemeinen Formel I gemäß den Ansprüchen 1 bis 6, **dadurch gekennzeichnet, daß**
a) eine Verbindung der allgemeinen Formel 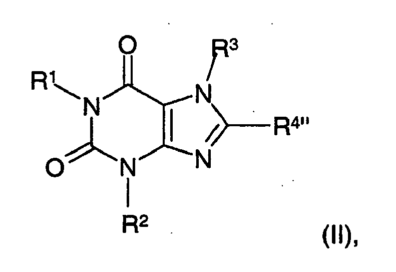 in der R¹, R² und R³ wie in Anspruch 1 erwähnt definiert sind und
R^{4'}' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe substituiert ist, entschützt wird,
b) eine Verbindung der allgemeinen Formel 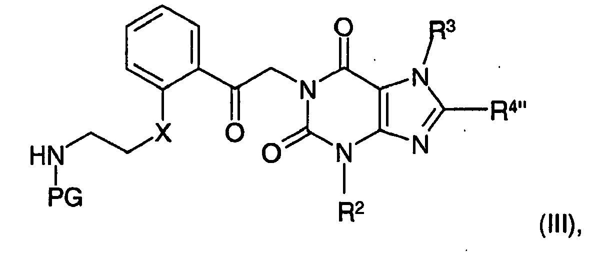 in der R² und R³ wie in Anspruch 1 erwähnt definiert sind,
R⁴" eine der eingangs für R⁴ erwähnten Gruppen bedeutet, die eine Imino-, Amino- oder Alkylaminogruppe enthalten, wobei die Imino-, Amino- bzw. Alkylaminogruppe durch eine der vorstehend erwähnten Schutzgruppe substituiert ist,
X ein Sauerstoff- oder Schwefelatom, eine Sulfinyl-, Sulfonyl- oder eine durch Rₓ substituierte Iminogruppe bedeutet, und
die -CH₂-CH₂-X-Phenyl-Einheit durch R¹⁰ bis R¹⁴ substituiert ist und zusätzlich durch eine C₁₋₃-Alkylgruppe substituiert sein kann,
wobei Rₓ und R¹⁰ bis R¹⁴ wie eingangs erwähnt definiert sind, und
PG ebenfalls eine der vorstehend erwähnten Schutzgruppen bedeutet,
wobei beide Schutzgruppen gleichzeitig oder nacheinander abspaltbar sind, entschützt und cyclisiert wird,
c) zur Herstellung einer Verbindung der allgemeinen Formel I, in der R¹ eine gegebenenfalls durch eine in den Ansprüchen 1 bis 5 definierte Gruppe substituierte 3-Oxo-2,3-dihydro-isoindol-1-ylidenmethylgruppe darstellt, eine Verbindung der allgemeinen Formel 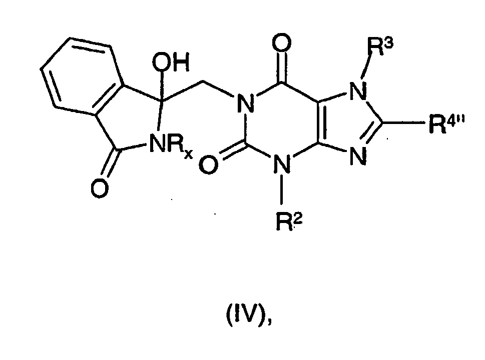 in der die Benzogruppe durch R¹⁰ bis R¹⁴ substituiert ist,
und R¹⁰ bis R¹⁴ sowie Rₓ, R² und R³ und wie eingangs erwähnt definiert sind, und R^{4'}' eine der eingangs für R⁴ erwähnten Gruppen bedeutet, die eine Imino-, Amino- oder Alkylaminogruppe enthalten, wobei die Imino-, Amino- bzw. Alkylaminogruppe durch eine der vorstehend erwähnten Schutzgruppe substituiert ist und
die Dehydratisierung unter den gleichen Reaktionsbedingungen wie die Abspaltung der Schutzgruppe erfolgt,
entschützt und dehydratisiert wird,
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.
